# EUROPEAN PATENT APPLICATION

(11) **EP 4 818 216 A1**
(43) Date of publication of application: **07.10.2026**
(21) Application number: 24897271.3
(22) Date of filing: 12.11.2024
(51) Int. Cl.: C08F 20/10, C07C 69/54, C07C 233/09, C07C 271/16, C07C 275/10, C08F 20/54, C09D 4/02, C09K 3/18

(54) **HYDROCARBON END GROUP-CONTAINING COMPOUND, CURABLE COMPOSITION FOR FORMING COATING FILM, CURED COATING FILM, AND ARTICLE**

(30) Priority: 30.11.2023 JP 2023202388
(71) Applicant: SHIN-ETSU CHEMICAL CO., LTD., Tokyo 100-0005 (JP)
(72) Inventor: MORI, Seiya, Annaka-shi, Gunma 379-0224 (JP); SAKANO, Yasunori, Annaka-shi, Gunma 379-0224 (JP); SAKOH, Ryusuke, Annaka-shi, Gunma 379-0224 (JP)
(74) Representative: Ter Meer und Partner mbB
(86) International application number: PCT/JP2024/040075
(87) International publication number: WO 2025/115584

(57) **Abstract**

According to the present invention, by adding a hydrocarbon end group-containing compound which is represented by formula (1) and does not contain a fluorine atom in the structure to a thermosetting composition or a composition that is curable by an active energy ray such as an ultraviolet ray or an electron beam, excellent water repellency, antifouling properties and wear resistance can be imparted to a cured coating film produced from the composition. 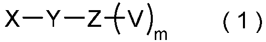 [X represents R¹O- or R²R³N-, R¹ represents a monovalent hydrocarbon group having 20 to 80 carbon atoms, R² represents a monovalent hydrocarbon group having 10 to 40 carbon atoms, R³ represents H or R², the total number of carbon atoms in R² and R³ is 20 to 80, Y represents a single bond or the following group

*-C(=O)-**

*-C(=O)-O-**

*-C(=O)-NR⁴-**

*-C(=S)-NR⁴-**

(wherein * denotes an atomic bond with X, ** denotes an atomic bond with Z, and R⁴ represents H or a monovalent hydrocarbon group),
Z represents a single bond or a hydrocarbon group which has a valence of 2 to 4 and may contain O, S, N, and Si, V represents a polymerizable carbon-carbon double bond-containing monovalent hydrocarbon group which may contain O and N, and m represents a number of 1 to 3.]

## Description

### TECHNICAL FIELD

This invention relates to a hydrocarbon end group-containing compound. More particularly, it relates to a compound containing a hydrocarbon end group, but not fluorine in its structure, which when blended in a UV, EB or actinic energy radiation-curable composition or heat-curable composition, is capable of imparting water repellent, antifouling, and wear resistant properties to a cured film of the composition; a film-forming curable composition comprising the hydrocarbon end group-containing compound; a cured film obtained by curing the film-forming curable composition; and an article having the cured film on the surface of a substrate.

### BACKGROUND ART

In the prior art, hard coat treatment is widely used as the means for protecting the surface of resin moldings. This treatment is to form a hard cured resin layer or hard coat layer on the surface of moldings to make the molding surface resistant to damage. As the material of the hard coat layer, heat-curable compositions or UV, EB or actinic energy radiation-curable compositions are often used.

Under the trends of enlarging the utilization field and increasing the added value of resin moldings, it is increasingly desired to enhance the function of cured resin layers or hard coat layers. One desire for function enhancement is to provide the hard coat layers with antifouling properties. This is to impart water repellent, oil repellent and other properties to the surface of hard coat layers such that the hard coat layers are stain resistant or even when stained, stain is easily removable.

The methods for imparting antifouling properties to hard coat layers include a method of forming a hard coat layer and coating and/or fixing a fluorinated antifouling agent to the surface of the hard coat layer and another method of adding a fluorinated curable component to a curable resin composition prior to curing, and coating and curing the composition to form a hard coat layer and impart antifouling properties at the same time. For example, Patent Document 1 (JP-A H06-211945) discloses that by adding a fluoroalkyl acrylate to an acrylic curable resin composition and curing the composition, a hard coat layer endowed with antifouling properties is prepared.

Making efforts to develop a variety of fluorinated compounds capable of imparting antifouling properties to curable resin compositions, the inventors proposed the photo-curable fluorinated compounds, for example, in Patent Document 2 (JP-A 2010-53114), Patent Document 3 (JP-A 2010-138112), and Patent Document 4 (JP-A 2010-285501).

On the other hand, fluorinated compounds as typified by perfluorooctanoic acid (PFOA) tend to be difficultly decomposable and highly accumulative in the natural world. In the recent years, a wide range of fluorinated compounds is categorized as per/polyfluoroalkyl compounds (PFASs). It is speculated that the limitations on the use, sales and discharge of fluorinated compounds are strengthened as the PFAS regulations. Recently, it is rather desired to develop fluorine-free, i.e., non-fluorinated antifouling agents. However, when these prior art non-fluorinated compounds are used in the above-mentioned applications, the resulting cured films are not satisfactory on the industrial or commercial level with respect to water repellent, antifouling, and wear resistant properties.

### PRIOR ART DOCUMENTS

### PATENT DOCUMENTS

| | |
|---|---|
| Patent Document 1: | JP-A H06-211945 |
| Patent Document 2: | JP-A 2010-53114 |
| Patent Document 3: | JP-A 2010-138112 |
| Patent Document 4: | JP-A 2010-285501 |

### SUMMARY OF INVENTION

### TECHNICAL PROBLEM

An object of the invention, which has been made under the above-mentioned circumstances, is to provide a compound containing a hydrocarbon end group, but not fluorine in its structure, which when blended in a UV, EB or actinic energy radiation-curable composition or heat-curable composition, is capable of imparting water repellent, antifouling, and wear resistant properties to a cured film of the composition; a film-forming curable composition comprising the hydrocarbon end group-containing compound; a cured film obtained by curing the film-forming curable composition; and an article having the cured film on the surface of a substrate.

### SOLUTION TO PROBLEM

Making extensive investigations to attain the above object, the inventors have found that a hydrocarbon end group-containing compound not containing fluorine in its structure, represented by the general formula (1) shown below, when blended in a UV, EB or actinic energy radiation-curable composition or heat-curable composition as a non-fluorinated antifoulant, is capable of imparting water repellent, antifouling, and wear resistant properties to a cured film of the composition. The invention is predicated on this finding.

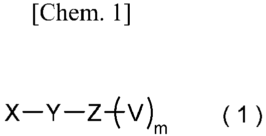

Herein X is R¹O- or R²R³N-, R¹ is a C₂₀-C₈₀ straight, branched or cyclic monovalent hydrocarbon group, R² is a C₁₀-C₄₀ straight, branched or cyclic monovalent hydrocarbon group, R³ is hydrogen or a C₁₀-C₄₀ straight, branched or cyclic monovalent hydrocarbon group, the total of carbon atoms in R² and R³ is from 20 to 80,

Y is a single bond or divalent organic group having the following structural formula:

*-C(=O)-**

*-C(=O)-O-**

*-C(=O)-NR⁴-**

*-C(=S)-NR⁴-**

wherein * designates a point of attachment to X in formula (1), ** designates a point of attachment to Z in formula (1), R⁴ is hydrogen or a C₁-C₈ straight, branched or cyclic monovalent hydrocarbon group,
Z is a single bond or C₁-C₂₀ di- to tetravalent hydrocarbon group which may contain at least one atom selected from oxygen, sulfur, nitrogen and silicon,
V is independently a C₂-C₂₀ monovalent hydrocarbon group which contains a polymerizable carbon-carbon double bond and which may contain oxygen and/or nitrogen, and
m is an integer of 1 to 3.

Accordingly, the invention provides a hydrocarbon end group-containing compound, a film-forming curable composition comprising the hydrocarbon end group-containing compound, a cured film obtained by curing the film-forming curable composition, and an article having the cured film on its surface.
[1] A compound which contains a hydrocarbon end group, but not fluorine in its structure, represented by the general formula (1): 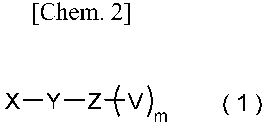 wherein X is R¹O- or R²R³N-, R¹ is a C₂₀-C₈₀ straight, branched or cyclic monovalent hydrocarbon group, R² is a C₁₀-C₄₀ straight, branched or cyclic monovalent hydrocarbon group, R³ is hydrogen or a C₁₀-C₄₀ straight, branched or cyclic monovalent hydrocarbon group, the total of carbon atoms in R² and R³ is from 20 to 80,
   Y is a single bond or divalent organic group having the following structural formula:

      *-C(=O)-**

      *-C(=O)-O-**

      *-C(=O)-NR⁴-**

      *-C(=S)-NR⁴-**

      wherein * designates a point of attachment to X in formula (1), ** designates a point of attachment to Z in formula (1), R⁴ is hydrogen or a C₁-C₈ straight, branched or cyclic monovalent hydrocarbon group,
   Z is a single bond or C₁-C₂₀ di- to tetravalent hydrocarbon group which may contain at least one atom selected from oxygen, sulfur, nitrogen and silicon,
   V is independently a C₂-C₂₀ monovalent hydrocarbon group which contains a polymerizable carbon-carbon double bond and which may contain oxygen and/or nitrogen, and
   m is an integer of 1 to 3.
[2] The hydrocarbon end group-containing compound of [1] wherein in formula (1), V has the following formula: 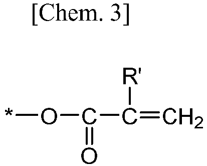 wherein * designates a point of attachment to Z in formula (1), and R' is hydrogen or a C₁-C₈ monovalent hydrocarbon group.
[3] The hydrocarbon end group-containing compound of [1] or [2] wherein in formula (1), Y has the following structural formula:

   *-C(=O)-NH-**

   wherein * designates a point of attachment to X in formula (1), and ** designates a point of attachment to Z in formula (1).
[4] The hydrocarbon end group-containing compound of any one of [1] to [3] wherein in formula (1), m is 1.
[5] The hydrocarbon end group-containing compound of any one of [1] to [4] wherein in formula (1), Z is a single bond or C₁-C₁₀ divalent hydrocarbon group which may contain oxygen and/or nitrogen.
[6] The hydrocarbon end group-containing compound of any one of [1] to [5] which is a hydrocarbon end group-containing acrylic compound represented by the general formula (2): 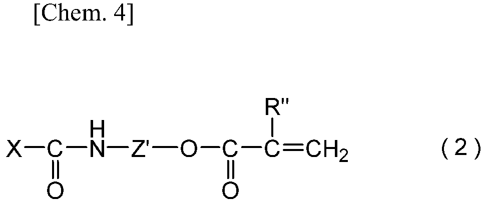 wherein X is as defined above, Z' is a C₁-C₁₀ divalent hydrocarbon group which may contain oxygen and/or nitrogen, and R" is hydrogen or methyl.
[7] The hydrocarbon end group-containing compound of any one of [1] to [5] which is a hydrocarbon end group-containing acrylic compound represented by the general formula (3): 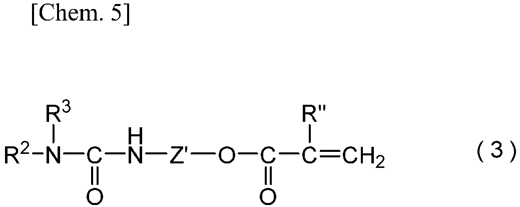 wherein R² and R³ are as defined above, Z' is a C₁-C₁₀ divalent hydrocarbon group which may contain oxygen and/or nitrogen, and R" is hydrogen or methyl.
[8] The hydrocarbon end group-containing compound of [7] wherein in formula (3), R² and R³ each are a C₁₀-C₄₀ straight monovalent hydrocarbon group, and R² and R³ have an equal number of carbon atoms.
[9] The hydrocarbon end group-containing compound of any one of [1] to [8], having a melting point of at least 20°C.
[10] A film-forming curable composition comprising a non-fluorinated hydrocarbon end group-containing compound having one C₂₀-C₈₀ hydrocarbon end group or two C₁₀-C₄₀ hydrocarbon end groups and at least one polymerizable group per molecule.
[11] The film-forming curable composition of [10] wherein the non-fluorinated hydrocarbon end group-containing compound is the hydrocarbon end group-containing compound of any one of [1] to [9].
[12] The film-forming curable composition of [10], further comprising a polymerization initiator.
[13] The film-forming curable composition of [12] which is of actinic energy radiation-curable type.
[14] The film-forming curable composition of any one of [10] to [13], further comprising a solvent.
[15] The film-forming curable composition of any one of [10] to [14], further comprising a non-fluorinated acrylic compound.
[16] The film-forming curable composition of any one of [10] to [15], comprising fluorine-free compounds.
[17] A cured film obtained by curing the film-forming curable composition of any one of [10] to [16].
[18] The cured film of [17], having a contact angle with water of at least 90° and a contact angle with oleic acid of at least 40° under conditions: droplet volume 2 µL, temperature 25°C, and relative humidity 40%.
[19] An article having the cured film of [17] or [18] on its surface.

### ADVANTAGEOUS EFFECTS OF INVENTION

A cured film obtained from a film-forming curable composition comprising the hydrocarbon end group-containing compound not containing fluorine in its structure is endowed with excellent water repellent, antifouling, and wear resistant properties. The hydrocarbon end group-containing compound is useful as an antifoulant for imparting water repellent, antifouling, and wear resistant properties to UV, EB or actinic energy radiation-curable hard coat or heat-curable hard coat agents, paint, resins, or antireflective coating compositions.

### DESCRIPTION OF EMBODIMENTS

As used herein, the "acrylic compound" collectively refers to compounds having an acryloyl group and α-substituted acryloyl group and encompasses compounds obtained by introducing two or more acryloyl or α-substituted acryloyl groups into polymers at side chains or ends by any desired method. Also, "(meth)acrylate" refers to either one or both of acrylate and methacrylate, "(meth)acryloyl group" refers to either one or both of acryloyl and methacryloyl groups, and "(meth)acrylic halide" refers to either one or both of acrylic halide and methacrylic halide.

The hydrocarbon end group-containing compound of the invention is a compound which contains a hydrocarbon end group, but not fluorine in its structure, represented by the general formula (1).

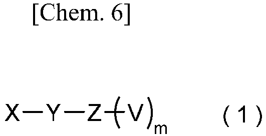

Herein X is R¹O- or R²R³N-, R¹ is a C₂₀-C₈₀ straight, branched or cyclic monovalent hydrocarbon group, R² is a C₁₀-C₄₀ straight, branched or cyclic monovalent hydrocarbon group, R³ is hydrogen or a C₁₀-C₄₀ straight, branched or cyclic monovalent hydrocarbon group, the total of carbon atoms in R² and R³ is from 20 to 80,
Y is a single bond or divalent organic group having the following structural formula:

   *-C(=O)-**

   *-C(=O)-O-**

   *-C(=O)-NR⁴-**

   *-C(=S)-NR⁴-**

   wherein * designates a point of attachment to X in formula (1), ** designates a point of attachment to Z in formula (1), R⁴ is hydrogen or a C₁-C₈ straight, branched or cyclic monovalent hydrocarbon group,
Z is a single bond or C₁-C₂₀ di- to tetravalent hydrocarbon group which may contain at least one atom selected from oxygen, sulfur, nitrogen and silicon,
V is independently a C₂-C₂₀ monovalent hydrocarbon group which contains a polymerizable carbon-carbon double bond and which may contain oxygen and/or nitrogen, and
m is an integer of 1 to 3.

The hydrocarbon end group-containing compound should contain a hydrocarbon chain end group having at least a predetermined carbon count and a polymerizable carbon-carbon double bond in its structure. The inclusion of a hydrocarbon chain end group of at least a predetermined carbon count featuring a close packing ability and a polymerizable carbon-carbon double bond serving as an actinic energy radiation-curable group or heat-curable group ensures that when the compound is blended in a film-forming curable composition and the composition is cured, the compound segregates on the surface of the cured film and forms (and is fixed as) a hard layer as a result of the hydrocarbon chain being packed. Then the cured film exhibits excellent water repellent, antifouling, and wear resistant properties. Although the compound does not segregate on the outermost surface in such cases as the compound is blended in combination with a certain component, the effect of improving the water repellency of the cured film itself is fully expectable. It is noted that the "packing ability" refers to the tendency that a plurality of hydrocarbon chains orient unidirectionally and closely on the surface of the cured film.

In formula (1), X is R¹O- or R²R³N-. R¹ is a C₂₀-C₈₀ straight, branched or cyclic monovalent hydrocarbon group, R² is a C₁₀-C₄₀ straight, branched or cyclic monovalent hydrocarbon group, and R³ is hydrogen or a C₁₀-C₄₀ straight, branched or cyclic monovalent hydrocarbon group. The total of carbon atoms in R² and R³ is from 20 to 80.

When the carbon count of R¹ is smaller than the upper limit, the compound is fully compatible with the film-forming curable composition. When the carbon count of R¹ is larger than the lower limit, the packing of hydrocarbon chains is closer. The advantage of R¹ as the water repellent and antifouling group is fully exerted.

When the total of carbon counts of R² and R³ is smaller than the upper limit, the compound is fully compatible with the film-forming curable composition. When the total of carbon counts of R² and R³ is larger than the lower limit, the packing of hydrocarbon chains is closer. The advantage of R² and R³ as the water repellent and antifouling group is fully exerted.

R¹ is preferably a C₂₁-C₆₀ straight or branched monovalent hydrocarbon group, more preferably a C₂₂-C₄₄ straight or branched monovalent hydrocarbon group.

Examples of R¹ are shown below.

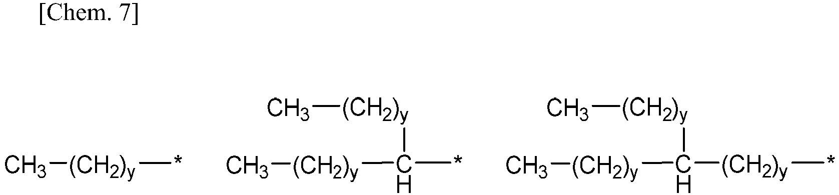

Herein * designates a point of attachment to the oxygen in X, y is independently such an integer of at least 1 that the total carbon count in each structure may be an integer of from 20 to 80, more preferably from 21 to 60, even more preferably from 22 to 44.

R² is preferably a C₁₁-C₃₀ straight or branched monovalent hydrocarbon group, more preferably a C₁₂-C₂₂ straight or branched monovalent hydrocarbon group. R³ is preferably hydrogen or a C₁₁-C₃₀ straight or branched monovalent hydrocarbon group, more preferably hydrogen or a C₁₂-C₂₂ straight or branched monovalent hydrocarbon group. The total carbon count in R² and R³ is preferably an integer of from 21 to 60, more preferably from 22 to 44.

Examples of R² are shown below.

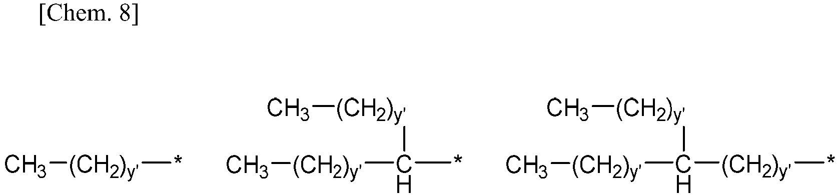

Herein * designates a point of attachment to the nitrogen in X, y' is independently an integer of at least 1, and the total carbon count in each structure is an integer of from 10 to 40, more preferably from 11 to 30, even more preferably from 12 to 22.

R³ is typically selected from hydrogen and the groups shown below.

[Chem. 9] CH₃-(CH₂)_{y'}-*

Herein * designates a point of attachment to the nitrogen in X, y' is independently an integer of at least 1, and the total carbon count in each structure is an integer of from 10 to 40, more preferably from 11 to 30, even more preferably from 12 to 22.

Examples of X are shown below.

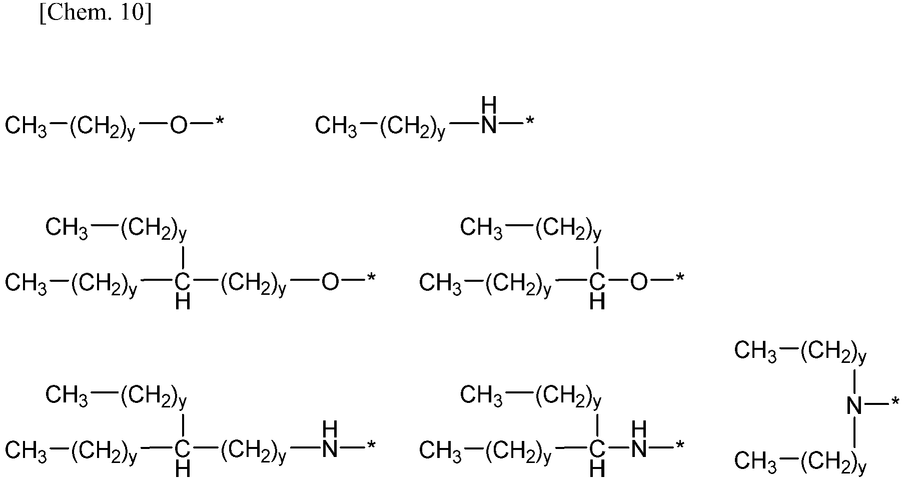

Herein * designates a point of attachment to Y in formula (1), y is independently an integer of at least 1, and the total carbon count in each structure is an integer of from 20 to 80, more preferably from 21 to 60, even more preferably from 22 to 44.

It is preferred that X have two CH₃ groups in its structure because excellent antifouling properties are developed. It is generally known that the surface of a solid having -CH₃ is more antifouling than the surface of a solid having only -CH₂- because of low surface free energy. The following structures are preferably used as X.

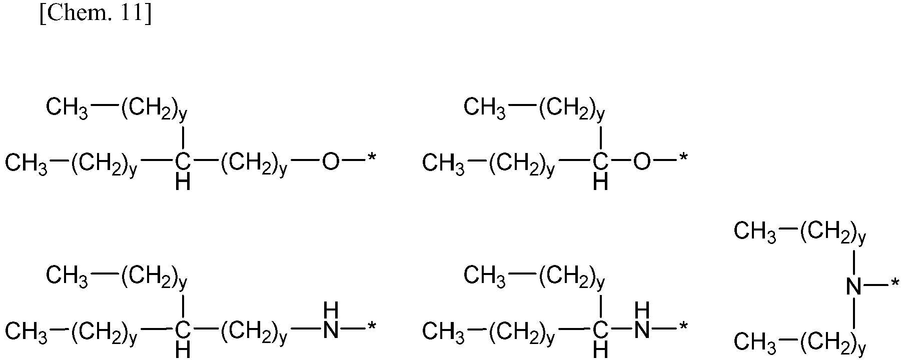

Herein * designates a point of attachment to Y in formula (1), y is independently an integer of at least 1, and the total carbon count in each structure is an integer of from 20 to 80, more preferably from 21 to 60, even more preferably from 22 to 44.

In formula (1), Y is a single bond or a divalent organic group having the following structural formula.

*-C(=O)-**

*-C(=O)-O-**

*-C(=O)-NR⁴-**

*-C(=S)-NR⁴-**

Herein * designates a point of attachment to X in formula (1), ** designates a point of attachment to Z in formula (1), and R⁴ is hydrogen or a C₁-C₈ straight, branched or cyclic monovalent hydrocarbon group.

R⁴ is preferably hydrogen or a C₁-C₈ straight, branched or cyclic monovalent hydrocarbon group, examples of which include alkyl groups such as methyl, ethyl, propyl, isopropyl, butyl, isobutyl, tert-butyl, pentyl, neopentyl, hexyl, cyclohexyl and octyl, alkenyl groups such as vinyl, allyl and propenyl, aryl groups such as phenyl, tolyl, xylyl and naphthyl, and aralkyl groups such as benzyl, phenylethyl and phenylpropyl. R⁴ is more preferably hydrogen.

Y is more preferably a group having the following structural formula:

*-C(=O)-NH-**

wherein * designates a point of attachment to X in formula (1), and ** designates a point of attachment to Z in formula (1). When Y is of the above structure, the compound is more compatible with the film-forming curable composition.

In formula (1), Z is a single bond or a C₁-C₂₀ di- to tetravalent hydrocarbon group which may contain at least one atom selected from oxygen, sulfur, nitrogen and silicon. Z is preferably a single bond when Y is a single bond.

Examples of the C₁-C₂₀ di- to tetravalent hydrocarbon group which may contain at least one atom selected from oxygen, sulfur, nitrogen and silicon include C₁-C₂₀ di- to tetravalent straight or branched hydrocarbon groups and C₁-C₂₀ di- to tetravalent hydrocarbon groups containing one or more moieties selected from ether, carbonyl (or ketone), ester, carbonate, -CH(OH)-, sulfinyl, sulfonyl, thioester, thiocarbonate, thiocarbamate, amino, amide, carbamate, urea, nitrogen-containing heterocyclic (e.g., oxazole, imidazole and triazole), silalkylene, silarylene, and straight, branched or cyclic organopolysiloxane moieties.

Z is preferably a single bond or a C₁-C₁₀ divalent hydrocarbon group which may contain oxygen and/or nitrogen. When Z is a divalent hydrocarbon group, the hydrocarbon end group-containing compound is a monoacrylic compound and more compatible with the film-forming curable composition.

The groups shown below are preferred as Z.

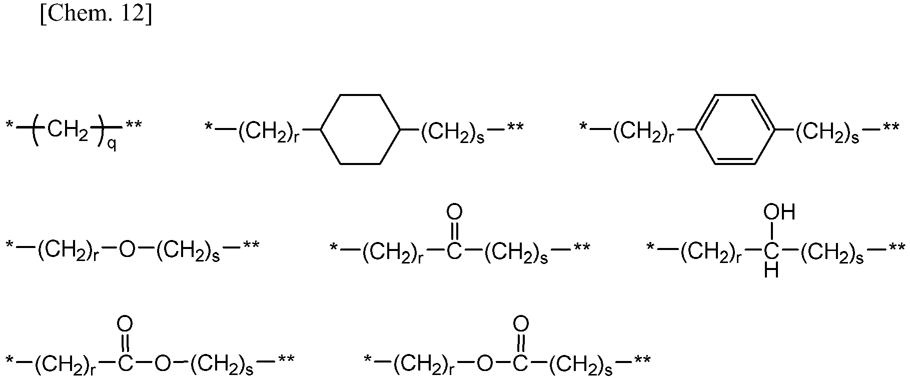

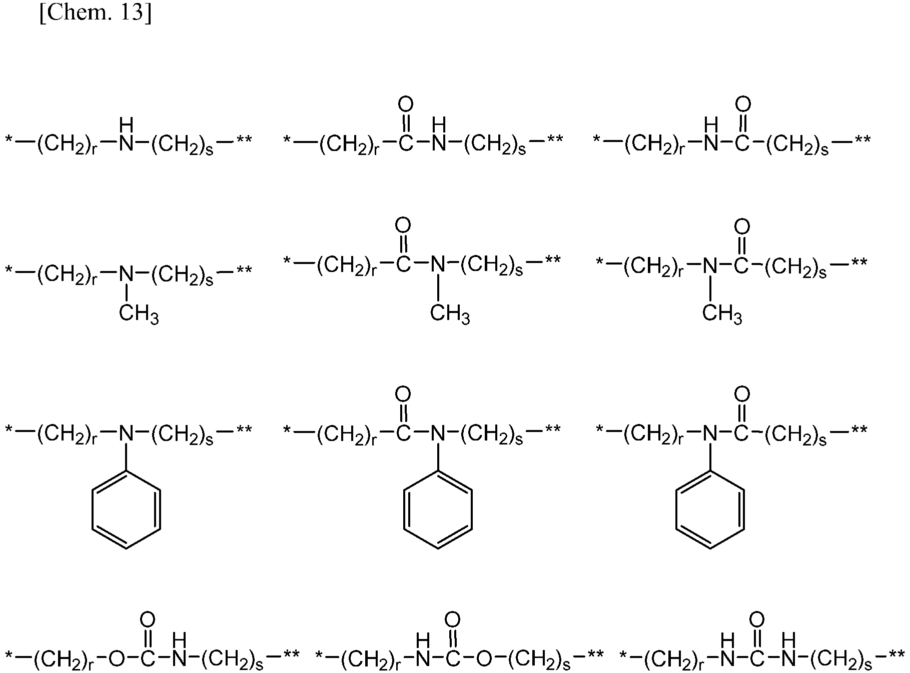

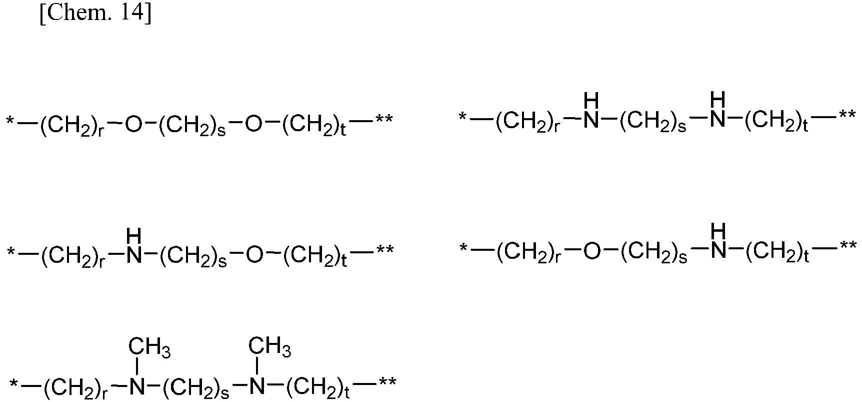

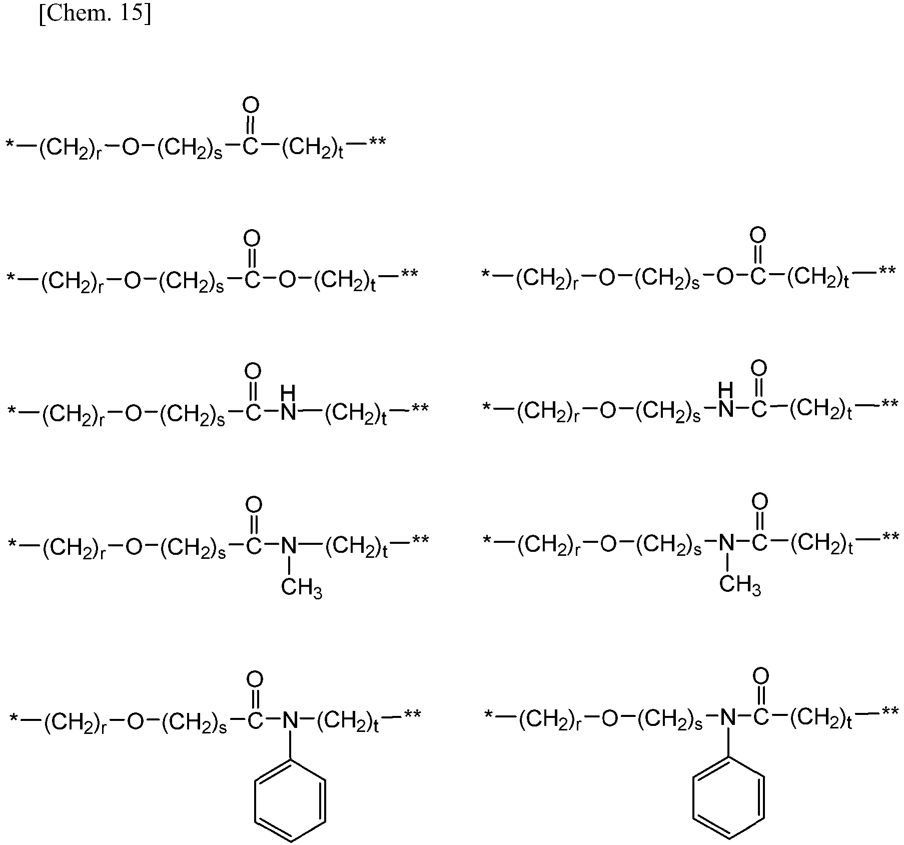

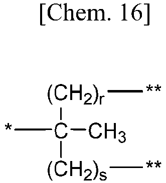

Herein * designates a point of attachment to Y in formula (1), ** designates a point of attachment to V in formula (1), q is an integer of 1 to 10, r, s and t each are an integer of 1 to 8, the sum of r and s is an integer of 2 to 10, and the sum of r, s and t is an integer of 3 to 10.

In formula (1), V is independently a C₂-C₂₀ monovalent hydrocarbon group which contains a polymerizable carbon-carbon double bond and which may contain oxygen and/or nitrogen. Examples of the polymerizable carbon-carbon double bond-containing group include acryloyl, α-substituted acryloyl, acryloxy, α-substituted acryloxy, acrylamide, α-substituted acrylamide, vinyl ether, cinnamate, and sorbate groups.

V is preferably an acryloyl, α-substituted acryloyl, acryloxy, or α-substituted acryloxy group of the following formula.

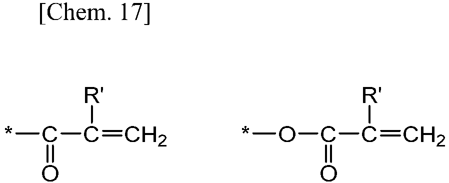

Herein * designates a point of attachment to Z in formula (1), and R' is hydrogen or a C₁-C₈ monovalent hydrocarbon group.

R' is hydrogen or a C₁-C₈ monovalent hydrocarbon group, examples of which include alkyl groups such as methyl, ethyl, propyl, isopropyl, butyl, isobutyl, tert-butyl, pentyl, neopentyl, hexyl, cyclohexyl and octyl, alkenyl groups such as vinyl, allyl and propenyl, aryl groups such as phenyl, tolyl, xylyl and naphthyl, and aralkyl groups such as benzyl, phenylethyl and phenylpropyl. R' is more preferably hydrogen or methyl.

V is preferably an acryloxy or α-substituted acryloxy group having the following formula.

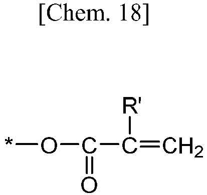

Herein * designates a point of attachment to Z in formula (1), and R' is as defined above.

The groups shown below are preferred as V.

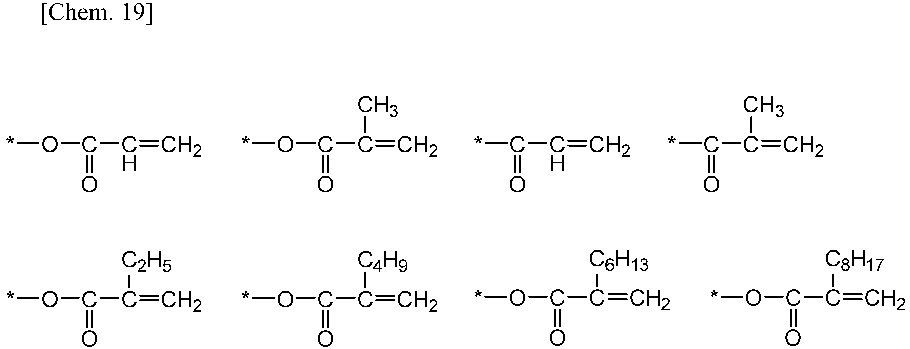

Herein * designates a point of attachment to Z in formula (1).

In formula (1), m is an integer of 1 to 3, preferably 1. If m is more than the upper limit, the proportion of hydrocarbon end group serving as a water repellent/antifouling group is relatively reduced, detracting from water repellent and antifouling properties.

The hydrocarbon end group-containing compound having formula (1) does not contain fluorine in its structure. Its tendency toward difficult decomposition and accumulation in the natural world is low as compared with conventional fluorinated compounds.

A series of hydrocarbon end group-containing compounds having formula (1) are obtained by changing the combination of X, Y, Z, and V in the formula.

Of the hydrocarbon end group-containing compounds having formula (1), hydrocarbon end group-containing acrylic compounds having the general formula (2) are more preferred.

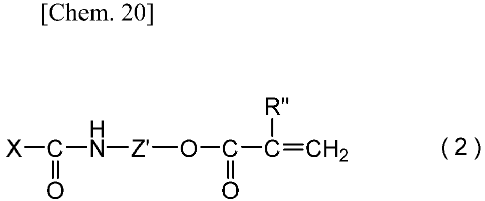

Herein X is as defined above, Z' is a C₁-C₁₀ divalent hydrocarbon group which may contain oxygen and/or nitrogen, and R" is hydrogen or methyl.

In formula (2), Z' is a C₁-C₁₀ divalent hydrocarbon group which may contain oxygen and/or nitrogen. Groups of the following formulae are preferably used as Z'.

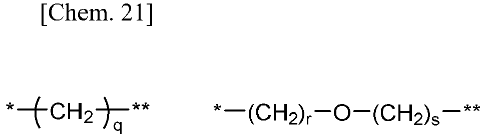

Herein * designates a point of attachment to the nitrogen atom in formula (2), ** designates a point of attachment to the oxygen atom in formula (2), q is an integer of 1 to 10, r and s each are an integer of 1 to 8, the sum of r and s is an integer of 2 to 10.

In the embodiment wherein the hydrocarbon end group-containing compound is a hydrocarbon end group-containing acrylic compound having formula (2), a cured film of a UV, EB or actinic energy radiation-curable composition or heat-curable composition to which the acrylic compound is added has more improved water repellent, antifouling, and wear resistant properties.

Especially preferred examples of the hydrocarbon end group-containing acrylic compound having formula (2) include those compounds of the following formulae.

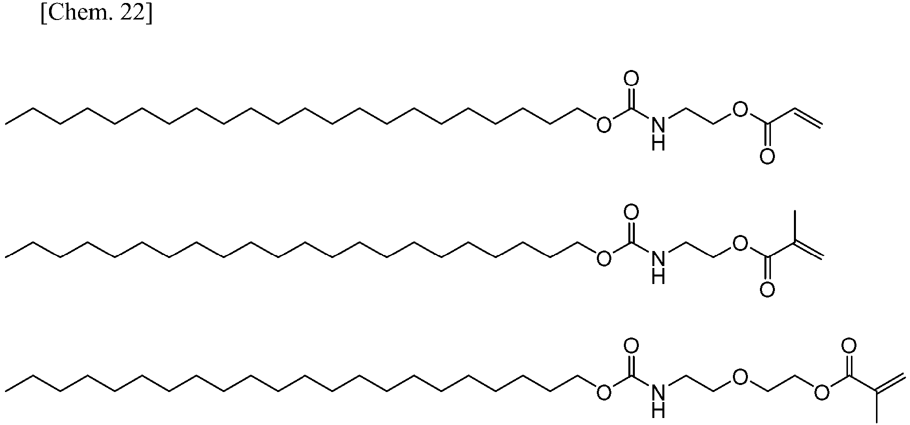

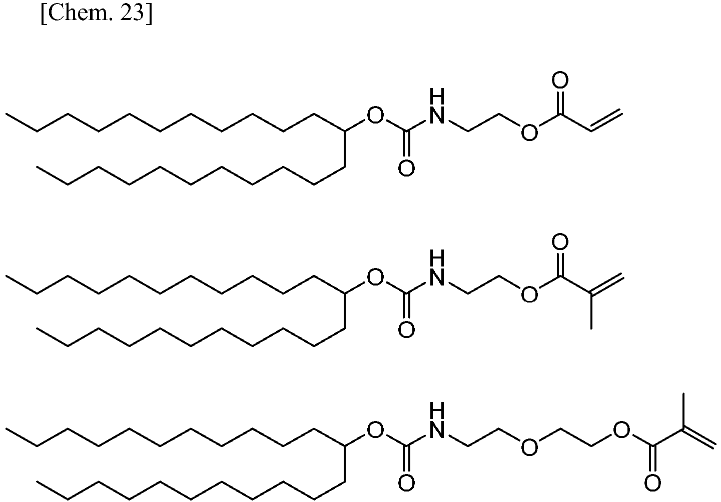

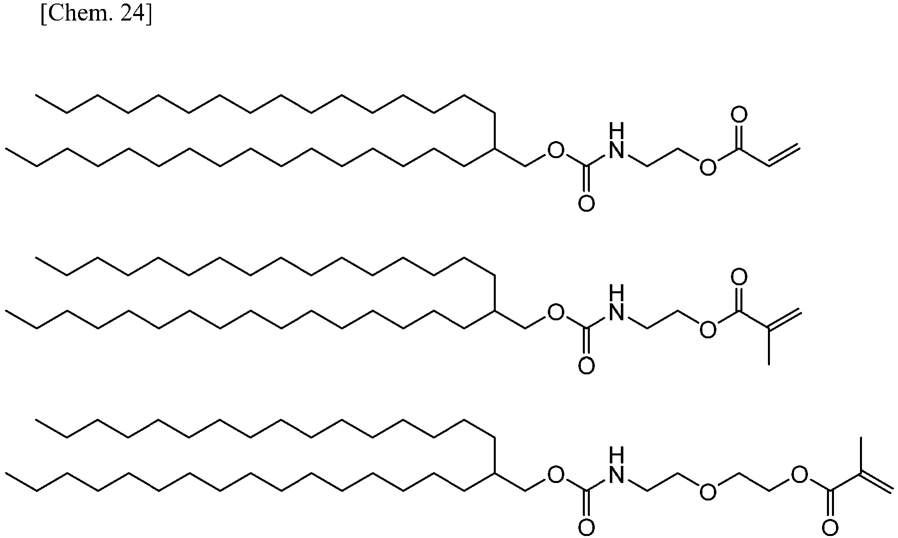

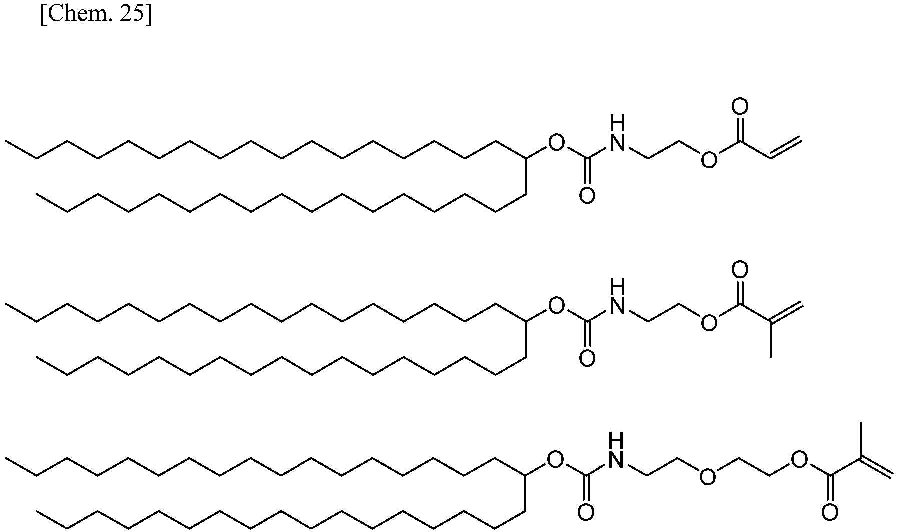

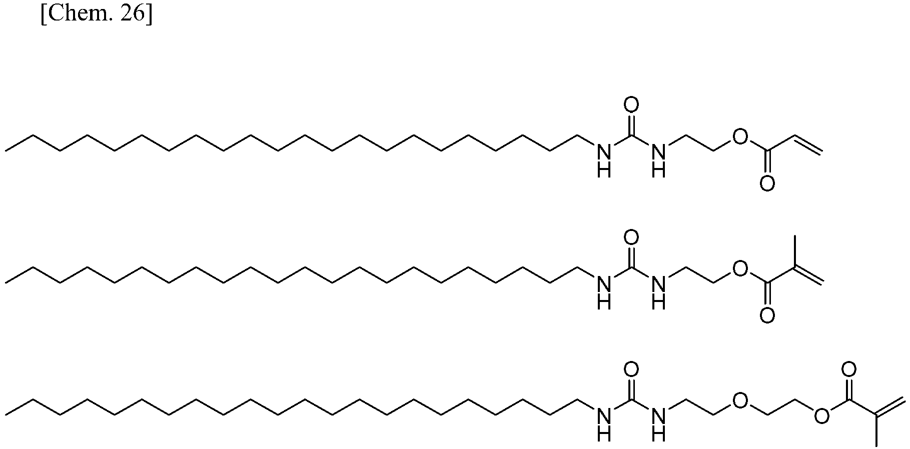

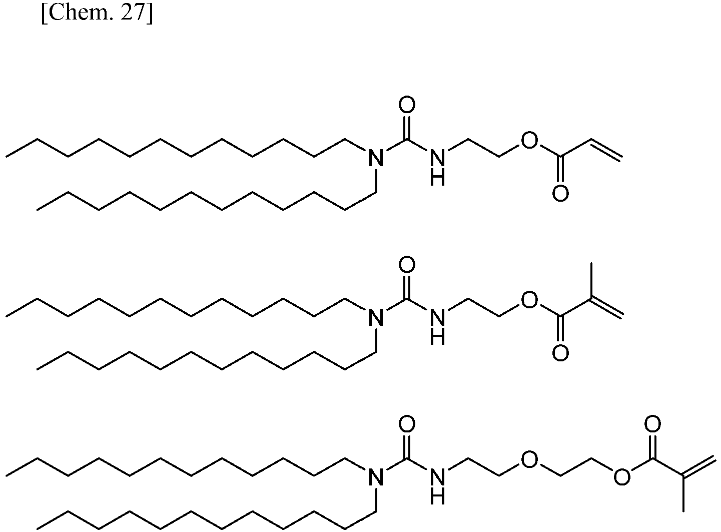

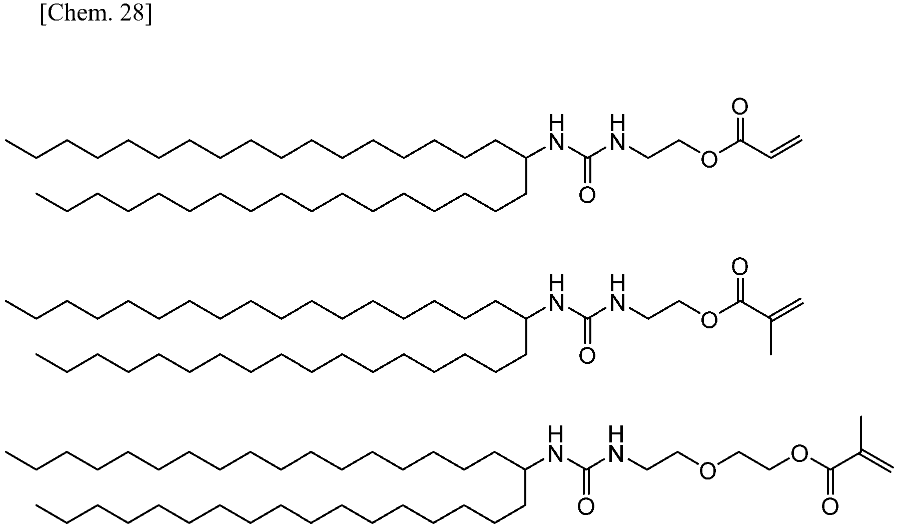

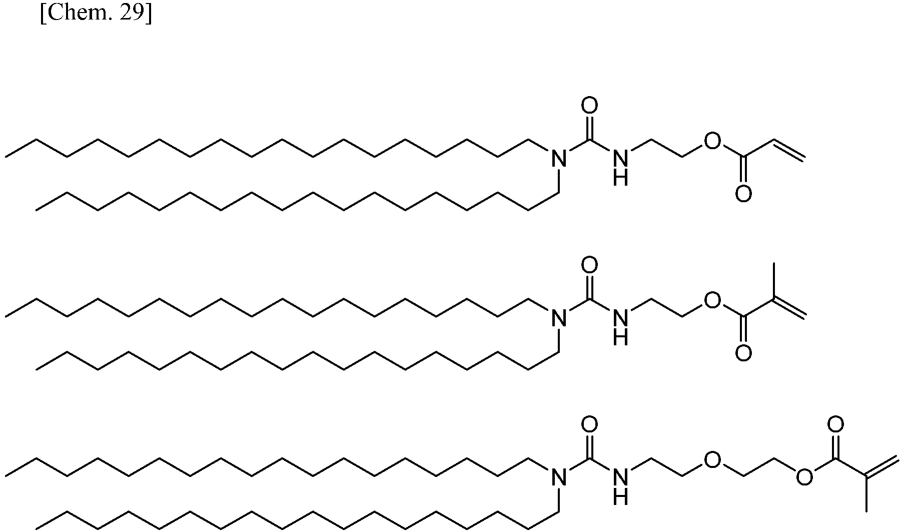

Of the hydrocarbon end group-containing compounds having formula (1), hydrocarbon end group-containing acrylic compounds having the general formula (3) are especially preferred.

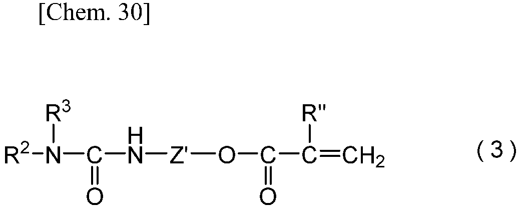

Herein R², R³, Z', and R" are as defined above.

In the embodiment wherein the hydrocarbon end group-containing compound is a hydrocarbon end group-containing acrylic compound having formula (3), a cured film of a UV, EB or actinic energy radiation-curable composition or heat-curable composition to which the acrylic compound is added has especially improved water repellent, antifouling, and wear resistant properties.

Especially preferred examples of the hydrocarbon end group-containing acrylic compound having formula (3) include those compounds of the following formulae.

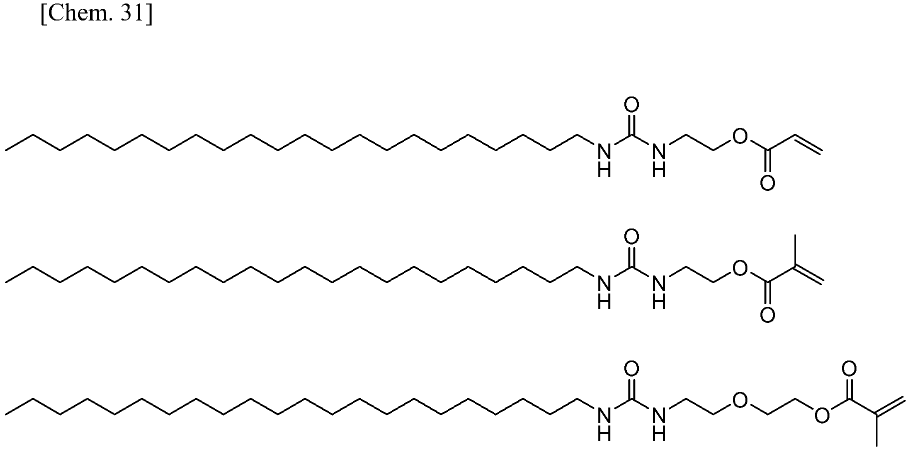

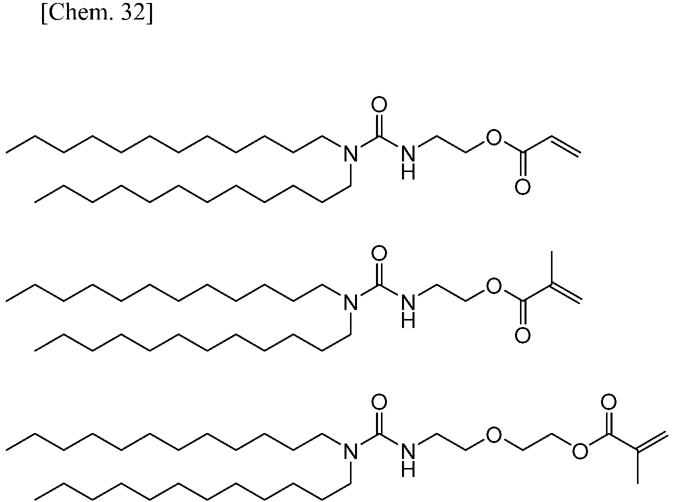

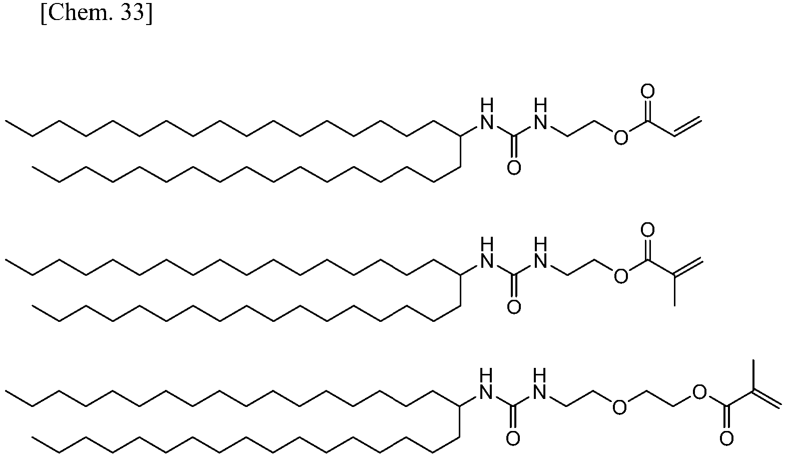

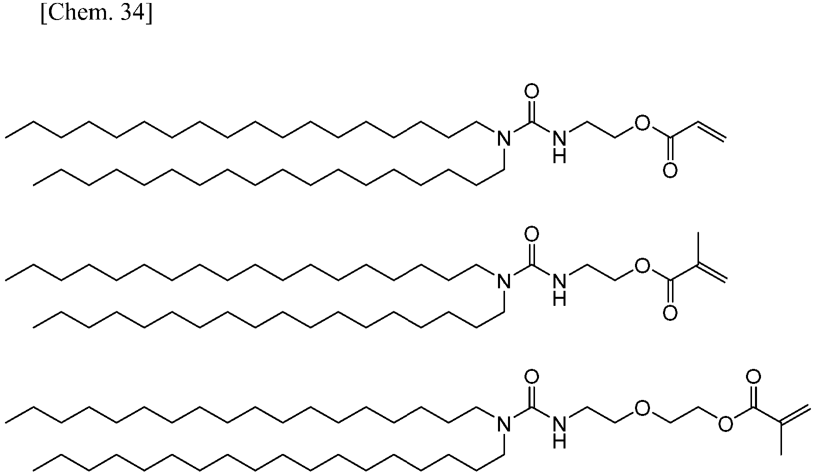

In formula (3), it is preferred that R² and R³ each be a C₁₀-C₄₀ straight monovalent hydrocarbon group, and R² and R³ have an equal carbon count. When R² and R³ satisfy these conditions, the distance between hydrocarbon groups is closer, the packing ability of hydrocarbon groups in the hydrocarbon end group-containing compound is further increased. Then a cured film of a UV, EB or actinic energy radiation-curable composition or heat-curable composition to which the compound is added has further improved wear resistance as a result of the compound forming a hard layer on the outermost surface.

Examples of the hydrocarbon end group-containing compound having formula (1) other than the examples of the hydrocarbon end group-containing acrylic compound having formula (2) and the hydrocarbon end group-containing acrylic compound having formula (3) include compounds having the following formulae.

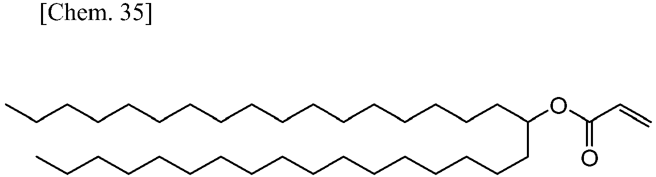

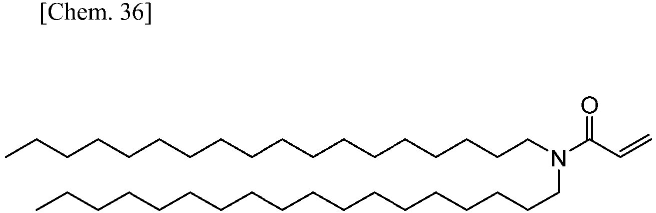

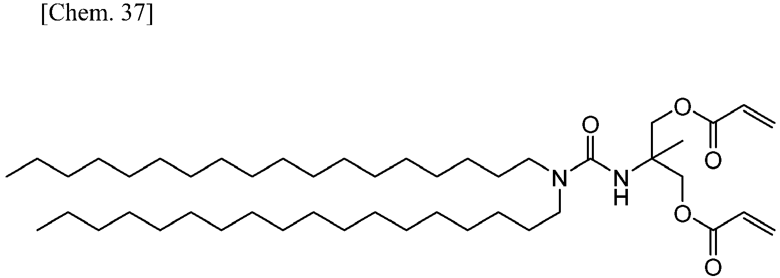

The hydrocarbon end group-containing compound having formula (1) preferably has a melting point of at least 20°C, more preferably 20 to 100°C. When the melting point is at least 20°C, a cured film of a UV, EB or actinic energy radiation-curable composition or heat-curable composition to which the compound is added has further improved wear resistance as a result of the compound forming a hard layer on the outermost surface.

As used herein, the melting point is measured under normal pressure by a differential scanning calorimeter (DSC). The measurement procedure is according to JIS K 7121. The measurement conditions include start temperature: -150°C, end temperature: 200°C, programming rate: 10°C/min, and ambient gas: nitrogen (flow rate: 50 ml/min).

In the embodiment wherein the hydrocarbon end group-containing compound having formula (1) is a hydrocarbon end group-containing acrylic compound having formula (2) or (3), the compound is prepared, for example, by the following method.

The hydrocarbon end group-containing acrylic compound can be prepared by mixing a hydrocarbon end group-containing alcohol or amine compound with a (meth)acryloyl group-containing isocyanate compound and conducting addition reaction.

The hydrocarbon end group-containing alcohol compound is represented by the formula (4). The hydrocarbon end group-containing amine compound is represented by the formula (5).

R¹OH (4)

R²R³NH (5)

Herein R¹, R² and R³ are as defined above.

Preferred examples of the alcohol compound having formula (4) are shown below.

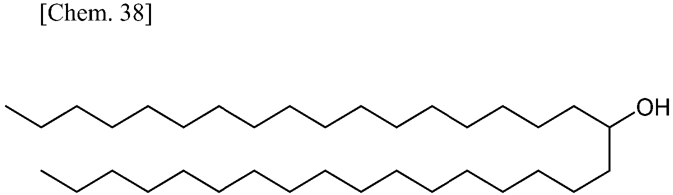

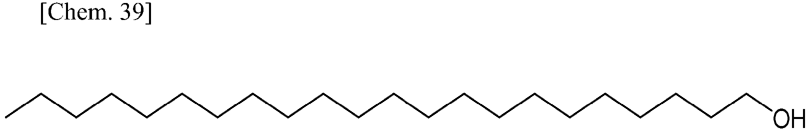

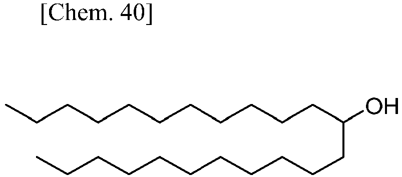

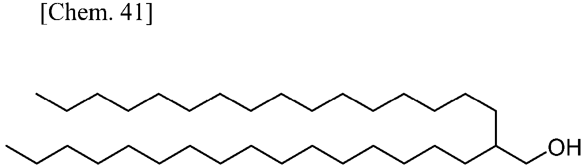

The hydrocarbon end group-containing alcohol compound may be prepared by hydride reduction of a hydrocarbon end group-containing aldehyde or ketone compound.

The following is exemplary of the hydrocarbon end group-containing aldehyde compound.

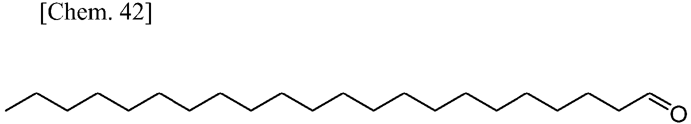

The following is exemplary of the hydrocarbon end group-containing ketone compound.

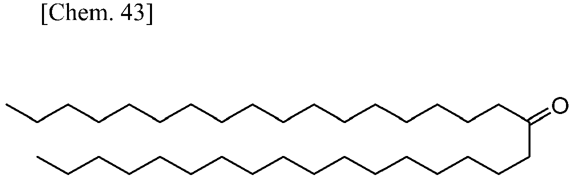

As the reducing agent used in the hydride reduction, sodium boron hydride and lithium aluminum hydride are preferred.

The reducing agent is preferably charged in an equimolar or more amount relative to the hydrocarbon end group-containing aldehyde or ketone compound so that the aldehyde or ketone compound may be reacted in its entirety. Specifically, the reducing agent is charged in an amount of preferably 1 to 5 equivalents, more preferably 1 to 3 equivalents per equivalent of the hydrocarbon end group-containing aldehyde or ketone compound in the reaction system.

The reaction may be performed in the system which is diluted with a suitable solvent, if necessary. The solvent used herein is not particularly limited as long as it is not reactive with the hydrocarbon end group-containing aldehyde or ketone compound and the reducing agent. Examples include hydrocarbon solvents such as petroleum benzine, toluene, xylene, hexane, cyclohexane, methylcyclohexane, ethylcyclohexane, heptane, octane (e.g., n-octane and isooctane,), and nonane (e.g., n-nonane and isononane); ketone solvents such as acetone, methyl ethyl ketone, methyl isobutyl ketone, cyclopentanone, and cyclohexanone; ether solvents such as tetrahydrofuran (THF), dipropyl ether, dibutyl ether, methyl cyclopentyl ether, methyl t-butyl ether, ethylene glycol dimethyl ether, diethylene glycol dimethyl ether, triethylene glycol dimethyl ether, and propylene glycol dimethyl ether; and alcohol solvents such as propylene glycol monomethyl ether, butanol, isopropanol, and methanol.

Although the amount of the solvent used is not particularly limited, the amount is preferably up to 20 times, more preferably up to 15 times the total weight of the reactants. If the amount of the solvent used is too much, sometimes the reaction rate is drastically reduced. When used, the amount of the solvent is preferably at least 0.5 time the total weight of the reactants.

The reaction may be performed at a temperature of -100°C to 100°C, preferably -50°C to 80°C for 5 minutes to 70 hours, preferably 30 minutes to 48 hours.

At the end of reaction, the unreacted reducing agent and the solvent are removed by such means as distillation, adsorption, filtration or washing, yielding the hydrocarbon end group-containing alcohol compound.

Preferred examples of the amine compound having formula (5) are shown below.

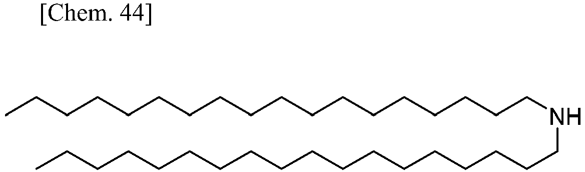

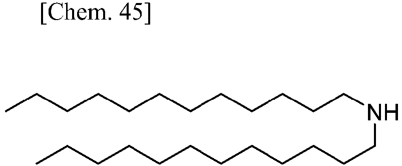

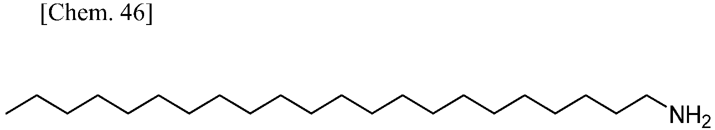

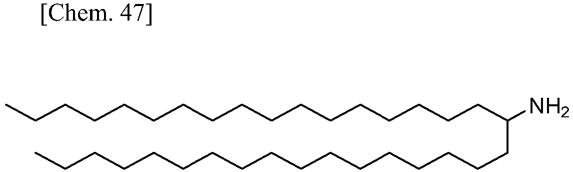

The (meth)acryloyl group-containing isocyanate compound is represented by the formula (6):

O=C=N-Z'-OC(=O)CR"=CH₂ (6)

wherein Z' and R" are as defined above.

Preferred examples of the (meth)acryloyl group-containing isocyanate compound are shown below.

O=C=N-CH₂CH₂-O-C(=O)-CH=CH₂

O=C=N-CH₂CH₂-O-C(=O)-C(CH₃)=CH₂

O=C=N-CH₂CH₂-O-CH₂CH₂-O-CH₂CH₂-O-C(=O)-C(CH₃)=CH₂

The (meth)acryloyl group-containing isocyanate compound is preferably charged in an equimolar or more amount relative to the total amount of active hydrogen in the hydrocarbon end group-containing alcohol or amine compound so that active hydrogen may be entirely reacted. Specifically, the amount of the (meth)acryloyl group-containing isocyanate compound is preferably from 1 to 2 equivalents, more preferably from 1 to 1.4 equivalents per equivalent of the hydrocarbon end group-containing alcohol or amine compound in the reaction system. If the amount of the (meth)acryloyl group-containing isocyanate compound is too much, it is difficult to remove the (meth)acryloyl group-containing isocyanate compound remaining at the end of reaction.

The reaction may be performed in the system which is diluted with a suitable solvent, if necessary. The solvent used herein is not particularly limited as long as it is not reactive with the hydroxy group in the hydrocarbon end group-containing alcohol or amine compound and the isocyanate group in the (meth)acryloyl group-containing isocyanate compound. Examples include hydrocarbon solvents such as petroleum benzine, toluene, xylene, hexane, cyclohexane, methylcyclohexane, ethylcyclohexane, heptane, octane (n-octane, isooctane, etc.), and nonane (n-nonane, isononane, etc.); ketone solvents such as acetone, methyl ethyl ketone, methyl isobutyl ketone, cyclopentanone, and cyclohexanone; ether solvents such as tetrahydrofuran (THF), dipropyl ether, dibutyl ether, methyl cyclopentyl ether, methyl t-butyl ether, ethylene glycol dimethyl ether, diethylene glycol dimethyl ether, triethylene glycol dimethyl ether, and propylene glycol dimethyl ether; alcohol solvents such as propylene glycol monomethyl ether, butanol, and isopropanol; and ester solvents such as ethyl acetate, propyl acetate, butyl acetate, pentyl acetate and propylene glycol monomethyl ether acetate. The solvent may be removed at the end of reaction by well-known means such as vacuum distillation, or the reaction solution may be used as diluted depending on the desired application.

Although the amount of the solvent used is not particularly limited, the amount is preferably up to 20 times, more preferably up to 15 times the total weight of the reactants. If the amount of the solvent used is too much, sometimes the reaction rate is drastically reduced. When used, the amount of the solvent is preferably at least 1 time the total weight of the reactants.

In the reaction, a polymerization inhibitor may be added if necessary. Although the polymerization inhibitor is not particularly limited, those compounds used as the polymerization inhibitor for acrylic compounds may be used. Examples of the inhibitor include hydroquinone, hydroquinone monomethyl ether, 4-tert-butyl catechol, and dibutyl hydroxytoluene.

The amount of the polymerization inhibitor used may be determined depending on the reaction conditions, purifying conditions after reaction, and final use conditions. The amount is preferably 0.01 to 5,000 ppm, more preferably 0.1 to 500 ppm based on the total weight of the reactants, though not particularly limited.

In the reaction, a suitable catalyst may be added if necessary. Suitable catalysts include alkyl tin ester compounds such as dibutyltin diacetate, dibutyltin dilaurate, dibutyltin dioctoate, dioctyltin diacetate, dioctyltin dilaurate, dioctyltin dioctate, and stannous dioctanoate; titanate esters or titanium chelates such as tetraisopropoxytitanium, tetra-n-butoxy titanium, tetrakis(2-ethylhexoxy)titanium (also named tetrakis(2-ethylhexyl) orthotitanate), dipropoxybis(acetylacetonato)titanium, and titanium isopropoxyoctylene glycol; zirconium tetraacetyl acetonate, zirconium tributoxymonoacetyl acetonate, zirconium monobutoxyacetyl acetonate bis(ethyl acetoacetate), zirconium dibutoxy bis(ethyl acetoacetate), zirconium tetraacetyl acetonate, and zirconium chelate compounds. The catalyst is not limited to a single compound and a mixture of two or more compounds may also be used.

The reaction rate can be accelerated by adding the catalyst in an amount of preferably 0.01 to 2% by weight, more preferably 0.05 to 1% by weight based on the total weight of the reactants.

The reaction is performed at a temperature of 0°C to 120°C, preferably 10°C to 70°C for 1 minute to 50 hours, preferably 10 minutes to 48 hours. If the reaction temperature is too low, the reaction rate may be too slow. If the reaction temperature is too high, polymerization of (meth)acryloyl groups can take place as side reaction.

At the end of reaction, the unreacted isocyanate compound and the solvent are removed by such means as distillation, adsorption, filtration or washing, yielding the hydrocarbon end group-containing acrylic compound having formula (2) or (3).

Upon termination of reaction, an alcohol compound such as methanol or ethanol may be added to the system to form a urethane bond with the unreacted isocyanate compound. The urethane (meth)acrylate thus formed may be removed by the same means as the unreacted isocyanate compound although the system in which the urethane (meth)acrylate remains may be used as such.

In the embodiment wherein the hydrocarbon end group-containing compound having formula (1) is a compound other than the hydrocarbon end group-containing acrylic compound having formula (2) or (3), the other compound is prepared, for example, by the following method.

For example, the hydrocarbon end group-containing acrylic compound may be obtained by reacting a hydrocarbon end group-containing alcohol or amine compound with a (meth)acrylic halide to form an ester. Examples of the hydrocarbon end group-containing alcohol or amine compound used herein are as exemplified above.

As the (meth)acrylic halide, acrylic chloride and methacrylic chloride are especially preferred.

The (meth)acrylic halide is preferably charged in an equimolar or more amount relative to the amount of the hydrocarbon end group-containing alcohol or amine compound so that the alcohol or amine compound may be reacted in its entirety. Specifically, the amount of the (meth)acrylic halide is preferably from 1 to 2 equivalents, more preferably from 1 to 1.8 equivalents per equivalent of the hydrocarbon end group-containing alcohol or amine compound in the reaction system.

The reaction may be performed in the system which is diluted with a suitable solvent, if necessary. The solvent used herein is not particularly limited as long as it is not reactive with the hydrocarbon end group-containing alcohol or amine compound and halogen in the (meth)acrylic halide. Examples include hydrocarbon solvents such as toluene, xylene, and isooctane; ether solvents such as tetrahydrofuran (THF), diisopropyl ether, and dibutyl ether; and ketone solvents such as acetone, methyl ethyl ketone, methyl butyl ketone, methyl isobutyl ketone, and cyclohexanone. The solvent may be removed at the end of reaction by well-known means such as vacuum distillation, or the reaction solution may be used as diluted depending on the desired application.

Although the amount of the solvent used is not particularly limited, the amount is preferably up to 20 times, more preferably up to 15 times the total weight of the reactants. If the amount of the solvent used is too much, sometimes the reaction rate is drastically reduced. When used, the amount of the solvent is preferably at least 1 time the total weight of the reactants.

In the reaction, a polymerization inhibitor may be added if necessary. Although the polymerization inhibitor is not particularly limited, those compounds used as the polymerization inhibitor for acrylic compounds may be used. Examples of the inhibitor include hydroquinone, hydroquinone monomethyl ether, 4-tert-butylcatechol, and dibutylhydroxytoluene.

The amount of the polymerization inhibitor used may be determined depending on the reaction conditions, purifying conditions after reaction, and final use conditions. The amount is preferably 0.01 to 5,000 ppm, more preferably 0.1 to 500 ppm based on the total weight of the reactants, though not particularly limited.

The esterification reaction may be carried out by combining the hydrocarbon end group-containing alcohol or amine compound with an acid acceptor, and optionally a solvent and a polymerization inhibitor, stirring them at 0°C to 100°C for 1 minute to 48 hours, and mixing the (meth)acrylic halide therewith.

As the acid acceptor, triethylamine, pyridine or urea may be used.

The amount of the acid acceptor used is desirably about 0.9 to 3 times the moles of the (meth)acrylic halide charged. If the amount is too small, more acid may be left untrapped. If the amount is too much, it is difficult to remove the excess of the acid acceptor.

After the (meth)acrylic halide is mixed, the reaction mixture is continuously stirred for 30 minutes to 10 hours while it is maintained at a temperature of 0 to 100°C. At the end of reaction, the unreacted (meth)acrylic halide, the salt resulting from reaction, and the reaction solvent are removed by such means as distillation, adsorption, filtration or washing, yielding the hydrocarbon end group-containing acrylic compound.

Upon termination of the reaction, the unreacted (meth)acrylic halide may be esterified by adding an alcohol compound such as methanol or ethanol to the reaction system. The thus formed (meth)acrylate ester may be removed by the same means as the removal of unreacted (meth)acrylic halide although the reaction system in which the (meth)acrylate ester remains may be used as such.

The hydrocarbon end group-containing compound having formula (1) obtained through the above-exemplified reaction may be used as a single substance after purifying/isolating operation like concentration, column purification, distillation or extraction. Also, the reaction solution may be used as a mixture containing the hydrocarbon end group-containing compound having formula (1). Alternatively, the reaction solution may be used after it is further diluted with an organic solvent.

A further embodiment of the invention is a film-forming curable composition comprising a non-fluorinated hydrocarbon end group-containing compound having one C₂₀-C₈₀ hydrocarbon end group or two C₁₀-C₄₀ hydrocarbon end groups and at least one polymerizable group per molecule. Preferably, the non-fluorinated hydrocarbon end group-containing compound is the hydrocarbon end group-containing compound not containing fluorine in its structure, represented by formula (1), alone or in admixture of two or more.

The amount of the non-fluorinated hydrocarbon end group-containing compound blended, specifically the hydrocarbon end group-containing compound of formula (1) blended is preferably from 0.005% to 99.9% by weight based on the overall components of the film-forming curable composition, exclusive of the solvent. Particularly when the composition is used in forming thick films (e.g., cured films of 0.5 to 100 µm thick), the amount of the non-fluorinated hydrocarbon end group-containing compound blended is preferably from 0.005% to less than 50% by weight based on the overall components of the film-forming curable composition, exclusive of the solvent. Particularly when the composition is used in forming thin films (e.g., cured films of 1 to 500 nm thick), the amount of the non-fluorinated hydrocarbon end group-containing compound blended is preferably from 50% to 99.9% by weight based on the overall components of the film-forming curable composition, exclusive of the solvent.

The film-forming curable composition preferably further comprises a polymerization initiator. In particular, a photopolymerization initiator is preferably used as the polymerization initiator because the film-forming curable composition can be of actinic energy radiation cure type.

The photopolymerization initiator is not particularly limited as long as it helps the non-fluorinated hydrocarbon end group-containing compound (and a non-fluorinated acrylic compound to be described later if any) cure upon UV irradiation. Preferred examples of the initiator include acetophenone, benzophenone, 2,2-dimethoxy-1,2-diphenylethan-1-one, 1-hydroxycyclohexyl phenyl ketone, 2-hydroxy-2-methyl-1-phenylpropan-1-one, 1-[4-(2-hydroxyethoxy)phenyl]-2-hydroxy-2-methyl-1-propan-1-one, 2-methyl-1-(4-methylthiophenyl)-2-morpholinopropan-1-one, 2-benzyl-2-dimethylamino-1-(4-morpholinophenyl)butanone-1, 2-dimethylamino-2-[(4-methylphenyl)methyl]-1-[4-(4-morpholinyl)phenyl]-1-butanone, 2,4,6-trimethylbenzoyl diphenylphosphine oxide, bis(2,4,6-trimethylbenzoyl)phenylphosphine oxide, 1,2-octanedione-1-[4-(phenylthio)-2-(O-benzoyloxime)], ethenone-1-[9-ethyl-6-(2-methylbenzoyl)-9H-carbazol-3-yl]-1-(O-acetyloxime), and 2-hydroxy-1-{4-[4-(2-hydroxy-2-methylpropionyl)benzyl]phenyl}-2-methylpropan-1-one, which may be used alone or in admixture of two or more.

The thermopolymerization initiator is not particularly limited as long as it helps the non-fluorinated hydrocarbon end group-containing compound (and a non-fluorinated acrylic compound to be described later if any) cure upon heating. Preferred examples of the initiator include diacyl peroxides, ketone peroxides, hydroperoxides, dialkyl peroxides, peroxy esters, azo compounds, and persulfates, which may be used alone or in admixture of two or more.

The amount of the polymerization initiator added may be determined as appropriate, depending on the curing conditions and the physical properties of a cured product of the film-forming curable composition. For example, the polymerization initiator is desirably used in an amount of 0.001 to 15 parts by weight, more desirably 0.01 to 10 parts by weight per 100 parts by weight of the total of non-volatiles in the film-forming curable composition exclusive of the solvent. An addition amount of less than the range may lead to under-cure whereas an addition amount of more than the range may largely affect the cured physical properties.

Preferably the film-forming curable composition further comprises a solvent. The curable composition containing the solvent has a reduced viscosity and is easy to handle.

The solvent used herein is desirably a non-fluorinated solvent. Examples include hydrocarbon solvents such as petroleum benzine, toluene, xylene, hexane, cyclohexane, methylcyclohexane, ethylcyclohexane, heptane, octane (e.g., n-octane and isooctane), and nonane (e.g., n-nonane and isononane); ketone solvents such as acetone, methyl ethyl ketone, methyl isobutyl ketone, cyclopentanone, and cyclohexanone; ether solvents such as tetrahydrofuran (THF), dipropyl ether, dibutyl ether, methyl cyclopentyl ether, methyl t-butyl ether, ethylene glycol dimethyl ether, diethylene glycol dimethyl ether, triethylene glycol dimethyl ether, and propylene glycol dimethyl ether; alcohol solvents such as propylene glycol monomethyl ether, butanol, and isopropanol; and ester solvents such as ethyl acetate, propyl acetate, butyl acetate, pentyl acetate, and propylene glycol monomethyl ether acetate.

Although the amount of the solvent used is not particularly limited, the solvent is preferably used in an amount of 1 to 1,000,000 parts by weight, more preferably 10 to 500,000 parts by weight per 100 parts by weight of the total of all components in the film-forming curable composition exclusive of the solvent.

The film-forming curable composition may further comprise other optional components, which are not particularly limited as long as a cured product is obtained by irradiating actinic energy radiation such as UV or EB or heating. When the film-forming curable composition is of actinic energy radiation cure type, a non-fluorinated acrylic compound (exclusive of the non-fluorinated hydrocarbon end group-containing compound) is preferably blended. When the non-fluorinated acrylic compound is blended, a cured film of the film-forming curable composition exhibits excellent film properties such as high hardness as well as liquid repellent, antifouling and wear resistant properties.

The non-fluorinated acrylic compound used herein may be either monofunctional or polyfunctional. An acrylic compound having two or more acryloyl groups per molecule is preferably blended.

The acrylic compound may be a compound having two or more acryloyl or α-substituted acryloyl groups per molecule. Examples include di- to hexa-functional or polyfunctional (meth)acrylic compounds such as 1,6-hexanediol di(meth)acrylate, neopentyl glycol di(meth)acrylate, ethylene glycol di(meth)acrylate, isocyanuric acid ethylene oxide-modified di(meth)acrylate, isocyanuric acid ethylene oxide-modified tri(meth)acrylate, trimethylolpropane tri(meth)acrylate, pentaerythritol tri(meth)acrylate, glycerol tri(meth)acrylate, tris(meth)acryloyloxyethyl phosphate, phthalic acid hydrogen-(2,2,2-tri(meth)acryloyloxymethyl)ethyl, glycerol tri(meth)acrylate, pentaerythritol tetra(meth)acrylate, ditrimethylolpropane tetra(meth)acrylate, dipentaerythritol penta(meth)acrylate, dipentaerythritol hexa(meth)acrylate, dipentaerythritol polyacrylate, and sorbitol hexa(meth)acrylate; the foregoing (meth)acrylic compounds modified with ethylene oxide, propylene oxide, epichlorohydrin, fatty acids, or alkyls; epoxy acrylates obtained by adding acrylic acid to epoxy resins; and copolymers obtained by introducing (meth)acryloyl groups to side chains of acrylate copolymers.

Also useful are urethane acrylates, reaction products of polyisocyanates with hydroxy-containing (meth)acrylates, reaction products of polyisocyanates and diol-terminated polyesters with hydroxy-containing (meth)acrylates, and reaction products obtained by reacting polyols with an excess of diisocyanates to form polyisocyanates and reacting the polyisocyanates with hydroxy-containing (meth)acrylates. Preferred are urethane acrylates obtained by reacting a hydroxy-containing (meth)acrylate selected from 2-hydroxyethyl (meth)acrylate, 2-hydroxy-3-acryloyloxypropyl (meth)acrylate and pentaerythritol tri(meth)acrylate, with a polyisocyanate selected from hexamethylene diisocyanate, isophorone diisocyanate, tolylene diisocyanate, lysin diisocyanate, norbornane diisocyanate, 1,3-bis(isocyanatomethyl)cyclohexane, methylene bis(4-cyclohexyl isocyanate), 2-methyl-1,3-diisocyanatocyclohexane, 2-methyl-1,5-diisocyanatocyclohexane, and diphenylmethane diisocyanate.

Also included are polyfunctional acrylic compounds having at least two acryloyl or α-substituted acryloyl groups per molecule, but free of urethane bond, and a mixture of at least two acrylic compounds including the aforementioned polyfunctional acrylic compounds and polyfunctional urethane acrylates having at least three acryloyl or α-substituted acryloyl groups per molecule, which are obtained by reacting an aliphatic polyisocyanate with a hydroxy-containing acrylic compound.

Examples of the polyfunctional acrylic compound having at least two acryloyl or α-substituted acryloyl groups per molecule, but free of urethane bond include trimethylolpropane tri(meth)acrylate, pentaerythritol tri(meth)acrylate, glycerol tri(meth)acrylate, pentaerythritol tetra(meth)acrylate, ditrimethylolpropane tetra(meth)acrylate, dipentaerythritol penta(meth)acrylate, dipentaerythritol hexa(meth)acrylate, sorbitol hexa(meth)acrylate, and compounds obtained by modifying the foregoing compounds with ethylene oxide or propylene oxide.

Examples of the polyfunctional urethane acrylate having at least three acryloyl or α-substituted acryloyl groups per molecule, which is obtained by reacting an aliphatic polyisocyanate with a hydroxy-containing acrylic compound, include products obtained by reacting a polyisocyanate having a functionality of at least 2 (which is obtained by reacting hexamethylene diisocyanate, norbornane diisocyanate, isophorone diisocyanate, trimers thereof, and di- or tri-functional isocyanates thereof, with an aliphatic diol, aliphatic polyol, and polyacrylate having a hydroxy group on side chain) with trimethylolpropane di(meth)acrylate, glycerin di(meth)acrylate, bis(2-(meth)acryloyloxyethyl)hydroxyethyl isocyanurate, pentaerythritol tri(meth)acrylate, ditrimethylolpropane tri(meth)acrylate, dipentaerythritol penta(meth)acrylate, and the foregoing compounds modified with ethylene oxide or propylene oxide; and products obtained by reacting an aliphatic polyol and a polyacrylate having a hydroxy group on side chain with an acrylic compound having an isocyanate group such as 2-isocyanatoethyl (meth)acrylate or 1,1-bis(acryloyloxymethyl)ethyl isocyanate.

Besides the foregoing compounds, microparticulate high polymers and microparticulate inorganic fillers which are modified on their surface with acryloyl groups are also included in the non-fluorinated acrylic compound.

The non-fluorinated acrylic compounds may be used alone or in admixture of plural compounds which are combined so as to facilitate coating step and enhance the properties of a cured film.

When used, the non-fluorinated acrylic compound is preferably blended in an amount of 20 to 1,000,000 parts by weight, more preferably 100 to 100,000 parts by weight per 100 parts by weight of the non-fluorinated hydrocarbon end group-containing compound.

The film-forming curable composition may further comprise any of thiol compounds, maleimide compounds, actinic energy radiation-reactive compounds excluding acryloyl group, polymerization inhibitors, antistatic agents, defoamers, viscosity regulators, photo-stabilizers, thermo-stabilizers, antioxidants, surfactants, colorants, and polymeric and inorganic fillers. These additives are not limited in structure, and any of well-known additives may be used as long as the objects of the invention are not compromised.

The film-forming curable composition may further contain unreacted reactants prior to the introduction of a reactive group of the hydrocarbon end group-containing compound having formula (1), and reaction intermediates.

Also, any existing compositions which are commercially marketed as an actinic energy radiation-curable composition or heat-curable composition having various additives blended therein from many manufacturers under the classification of paint, ink and hard coating agents may be used as part or the entirety of the film-forming curable composition. Even when such a commercial hard coating agent is used, any of polymerization inhibitors, antistatic agents, defoamers, viscosity regulators, photo-stabilizers, thermo-stabilizers, antioxidants, surfactants, colorants, and fillers may be additionally blended depending on a particular purpose.

Since it is recently speculated that the regulations on the use, sales and discharge of fluorinated compounds are strengthened as part of the PFAS control, the film-forming curable composition of the invention is preferably composed of fluorine-free compounds (that is, fluorinated components are not blended).

From the thus formulated film-forming curable composition of the invention, which comprises a hydrocarbon end group-containing compound of specific structure containing a hydrocarbon end group having a close packing ability and serving as a water repellent and antifouling group and a polymerizable carbon-carbon double bond serving as an actinic energy radiation-curable group or heat-curable group, a cured film having excellent water repellent, antifouling and wear resistant properties is obtained.

In a further aspect, the invention provides a cured film obtained by coating the film-forming curable composition onto a substrate surface and curing the composition, and an article having the cured film on its surface. Using the film-forming curable composition, a cured film (or cured resin layer) having excellent surface properties can be formed on the surface of a substrate as mentioned above. In particular, the film-forming curable composition is useful in imparting water repellent, antifouling and wear resistant properties to the surface of an acrylic hard coat. Then the substrate or article is endowed with a hard coat surface which is unsusceptible to staining with human fats like fingerprints, sebum and perspiration and cosmetics and is easy to wipe off such stains. Therefore, the film-forming curable composition is advantageously used in the application of a coating or protective film to the surface of a substrate or article which can be stained with human fats and cosmetics because of possible contact with the human body.

The cured film (or cured resin layer) formed using the film-forming curable composition can impart the desired properties to various articles (for example, articles having a surface substrate of paper, fabrics, metals, metal oxides, glass, plastics, ceramics, and quartz) by directly coating or evaporating and curing the composition to the surface of the intended article, or by coating or evaporating and curing the composition onto various substrate films (e.g., films of polyethylene, polypropylene, polyethylene terephthalate, polyethylene naphthalate, vinyl chloride, polystyrene, acrylic resin, polycarbonate, polyphenylene sulfide, polyether ether ketone, polyether sulfone, aramide, and polyimide) and attaching the film to the surface of the intended article.

The method of coating the film-forming curable composition is not particularly limited. Any of well-known coating methods such as roll coating, gravure coating, flow coating, dip coating, spray coating, spin coating, bar coating and screen printing may be used. The evaporation method, which is not particularly limited, may be either resistance heating or electron beam heating. After the composition is coated, it is cured by irradiating actinic energy radiation or heating.

When the coating is cured by irradiating actinic energy radiation, the radiation used may be electron beam, ultraviolet radiation (UV) or the like, preferably UV. The UV source is preferably a mercury lamp, metal halide lamp, or LED lamp. The dose of UV is desirably in the range of 10 to 10,000 mJ/cm², more desirably 20 to 4,000 mJ/cm² because a too low dose may allow some components to be left uncured and a too high dose may cause to degrade the coating and substrate. Also, to prevent oxygen cure inhibition, the atmosphere of UV irradiation may be purged with oxygen molecule-free inert gas such as nitrogen, carbon dioxide or argon; the coating surface may be covered with a UV-transmissive protective layer having parting properties and UV be irradiated from above the protective layer; or if the substrate is UV-transmissive, once the coating surface is covered with a protective layer having parting properties, UV may be irradiated from the surface of the substrate opposite to the coated surface. For effectively leveling the coating, the coating and substrate may be heated by any desired means such as a hot-air dryer before or during UV irradiation.

When the coating is cured by heating, the heat source used may be an oven, hot plate or the like. The heating conditions include 40 to 200°C, especially 50 to 150°C and 30 minutes to 36 hours, especially 1 to 24 hours.

The thickness of the cured film (or cured resin layer) formed using the film-forming curable composition is not particularly limited because the thickness largely varies the use method or the like.

For example, when the film-forming curable composition contains a large amount of the non-fluorinated acrylic compound, specifically when the amount of the non-fluorinated hydrocarbon end group-containing compound is 0.005 part to less than 50 parts by weight per 100 parts by weight of the entire components of the curable composition exclusive of the solvent, the cured film preferably has a thickness of 0.5 to 100 µm. When the film-forming curable composition contains a large amount of the non-fluorinated hydrocarbon end group-containing compound, specifically when the amount of the non-fluorinated hydrocarbon end group-containing compound is 50 parts to 99.9 parts by weight per 100 parts by weight of the entire components of the curable composition exclusive of the solvent, the cured film preferably has a thickness of 1 to 500 nm.

It is noted that the film thickness is measured by a thin film thickness measuring instrument base on optical interference (e.g., optical interference film thickness meter or reflectance spectroscopic film thickness meter) or thin film thickness measuring instrument based on spectroscopic ellipsometry (e.g., spectral ellipsometer).

The cured film (or cured resin layer) formed using the film-forming curable composition desirably has a contact angle with water of at least 90°, preferably at least 95° and a contact angle with oleic acid of at least 40°, preferably at least 45° at temperature 25°C and relative humidity 40%. It is noted that the contact angle is measured by a contact angle meter Drop Master (Kyowa Interface Science Co., Ltd.) under condition: droplet volume 2 µL. Preferably the film-forming curable composition is uniformly mixed in order to gain a contact angle in the range.

The inventive article has the cured film formed using the film-forming curable composition on the substrate surface, the cured film functions as a coating and surface protecting film on the article.

Exemplary articles include casings of various portable equipment such as tablet computers, mobile communication or information terminals such as cellular phones and smartphones, laptop computers, digital media players, watch or eyeglass form wearable computers, digital cameras, digital video cameras, and electronic book readers; displaying or operating surfaces of instruments like flat panel displays such as liquid crystal displays, plasma displays, organic EL displays, back projection displays, variable frequency drives (VFD), field emission projection displays, CRTs, and toner based displays and TVs, automobile exteriors, glossy surfaces of piano and furniture, surfaces of building stones such as marble, decorative building members in bath, toilet and lavatory rooms, transparent glass members, plastic members (e.g., acrylic resins and polycarbonate) and mirrors, such as protective glass for art object exhibition, show windows, show cases, photo frame covers, watches, windowpane of automobiles, trains and aircraft, automobile headlights and taillights.

Included are displaying inputs for various instruments having displaying inputs which are manipulated with fingers or palms like touch-panels, for example, tablet computers, laptop computers, watch type wearable computers, activity meters, mobile communication or information terminals such as cellular phones and smartphones, digital media players, electronic book readers, digital photo frames, game players, game player controllers, digital cameras, digital video cameras, car navigation systems, automatic teller machines, automatic venders, digital signages, security system terminals, POS terminals, and various controllers like remote controllers, panel switches of automobile-mounted instruments.

Other examples of the inventive article include optical recoding media such as opto-magnetic disks and optical disks, eyeglass lenses, camera lenses, projector lens prisms, lens seats, pellicle membranes, polarizers, optical filters, lenticular lenses, Fresnel lenses, antireflective coatings, optical parts and devices like optical fibers and optical couplers, and protective parts for the foregoing instruments.

### EXAMPLES

Synthesis Examples, Comparative Synthesis Examples, Examples, and Comparative Examples are given below for further illustrating the invention although the invention is not limited by Examples. In the following examples, room temperature is 25°C and normal pressure is atmospheric pressure. The melting point is measured under normal pressure by a differential scanning calorimeter (DSC). The measurement procedure is according to JIS K 7121. The measurement conditions include start temperature: -150°C, end temperature: 200°C, programming rate: 10°C/min, and ambient gas: nitrogen (flow rate: 50 ml/min). The film thickness is measured by a reflectance spectroscopic film thickness meter (measurement range 400 to 800 nm).

### [Synthesis Example 1] Synthesis of compound (b)

In a reactor, 100.00 g (1.97×10⁻¹ mol) of a compound having the formula (a): 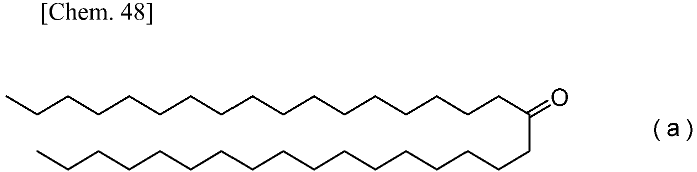 was mixed with 100.0 g of methanol and stirring was continued in nitrogen atmosphere at room temperature for 1 hour. Then 14.91 g (3.94×10⁻¹ mol) of sodium boron hydride was added to the reaction system, which was stirred in nitrogen atmosphere at room temperature for 12 hours. The solvent and unreacted components were then removed by water washing and distillation under reduced pressure, obtaining 91.21 g of the product.

By ¹H-NMR spectroscopy, the product was identified to be a compound having the formula (b).

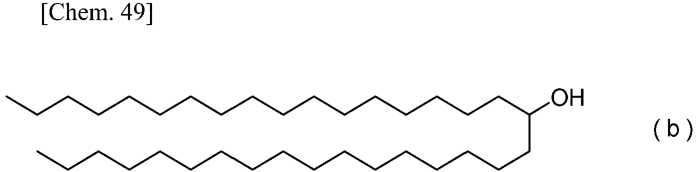

### [Synthesis Example 2] Synthesis of hydrocarbon end group-containing compound (A)

In a reactor, 10.00 g (1.96×10⁻² mol) of the compound (obtained in Synthesis Example 1) having the formula (b): 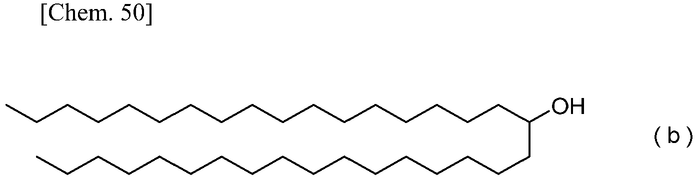 was mixed with 100.0 g of THF and 2.97 g (2.94×10⁻² mol) of triethylamine, which was stirred in nitrogen atmosphere at 40°C for 1 hour. Then 2.66 g (2.94×10⁻² mol) of acrylic chloride was added to the system, which was stirred in nitrogen atmosphere at 40°C for 6 hours. The solvent, by-products and unreacted components were then removed by water washing and distillation under reduced pressure, obtaining 8.78 g of the product.

By ¹H-NMR spectroscopy, the product was identified to be a compound having the formula (A). The compound had a melting point of 28°C under normal pressure.

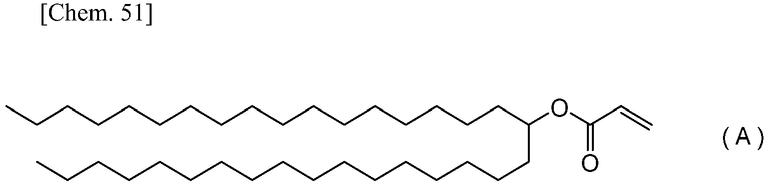

### [Synthesis Example 3] Synthesis of hydrocarbon end group-containing compound (B)

In a reactor, 10.00 g (1.92×10⁻² mol) of a compound having the formula (c): 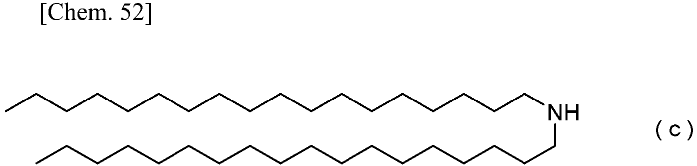 was mixed with 100.0 g of THF and 2.91 g (2.88×10⁻² mol) of triethylamine, which was stirred in nitrogen atmosphere at 40°C for 1 hour. Then 2.61 g (2.88×10⁻² mol) of acrylic chloride was added to the system, which was stirred in nitrogen atmosphere at 40°C for 6 hours. The solvent, by-products and unreacted components were then removed by water washing and distillation under reduced pressure, obtaining 8.62 g of the product.

By ¹H-NMR spectroscopy, the compound was identified to have a structure of the formula (B). The compound had a melting point of 32°C under normal pressure.

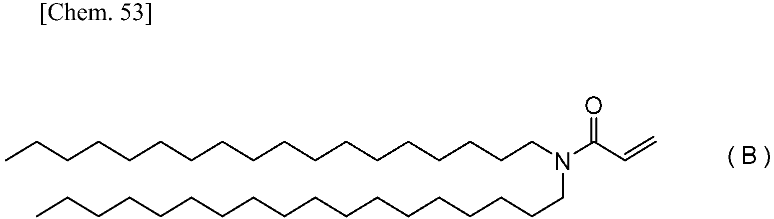

### [Synthesis Example 4] Synthesis of hydrocarbon end group-containing compound (C)

In a reactor, 10.00 g (1.96×10⁻² mol) of the compound (obtained in Synthesis Example 1) having the formula (b): 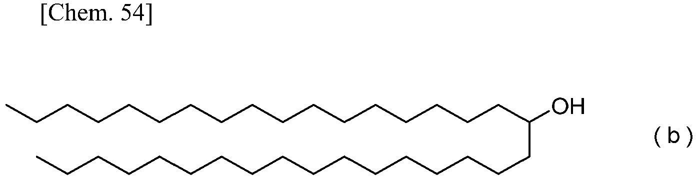 was mixed with 3.04 g (2.15×10⁻² mol) of 2-isocyanatoethyl acrylate, 50.00 g of THF and 0.03 g of tetrakis(2-ethylhexyl) orthotitanate, which was aged at 50°C for 12 hours. The solvent and unreacted components were then distilled off under reduced pressure, obtaining 12.88 g of the product.

By ¹H-NMR spectroscopy, the compound was identified to have a structure of the formula (C). The compound had a melting point of 58°C under normal pressure.

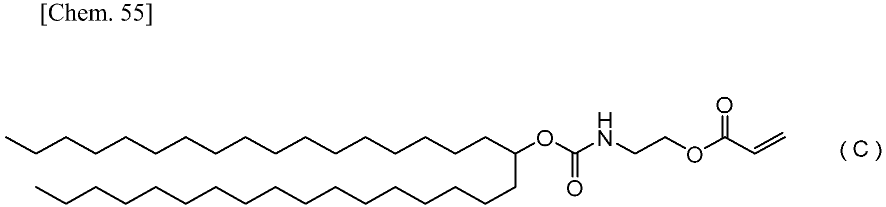

### [Synthesis Example 5] Synthesis of hydrocarbon end group-containing compound (D)

In a reactor, 10.00 g (3.06×10⁻² mol) of a compound having the formula (d): 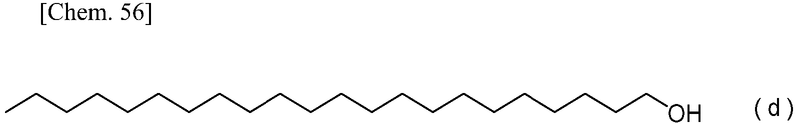 was mixed with 4.75 g (3.37×10⁻² mol) of 2-isocyanatoethyl acrylate, 50.00 g of THF and 0.03 g of tetrakis(2-ethylhexyl) orthotitanate, which was aged at 50°C for 12 hours. The solvent and unreacted components were then distilled off under reduced pressure, obtaining 14.40 g of the product.

By ¹H-NMR spectroscopy, the compound was identified to have a structure of the formula (D). The compound had a melting point of 34°C under normal pressure.

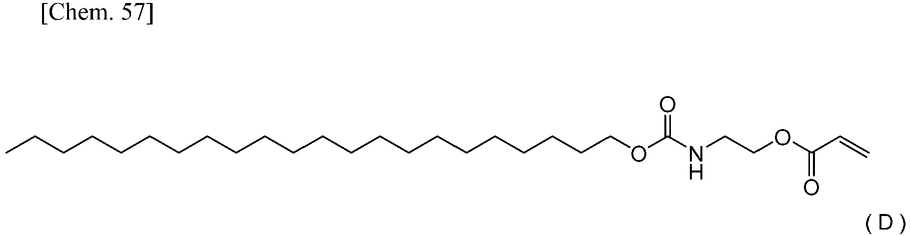

### [Synthesis Example 6] Synthesis of hydrocarbon end group-containing compound (E)

In a reactor, 10.00 g (1.96×10⁻² mol) of the compound (obtained in Synthesis Example 1) having the formula (b): 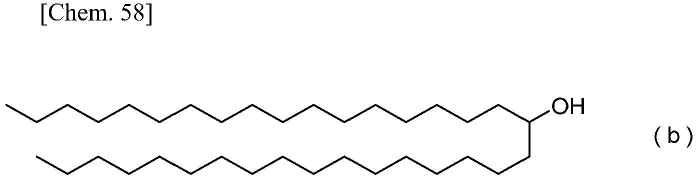 was mixed with 4.29 g (2.15×10⁻² mol) of 2-(2-methacryloyloxyethyloxy)ethyl isocyanate, 50.00 g of THF and 0.03 g of tetrakis(2-ethylhexyl) orthotitanate, which was aged at 50°C for 12 hours. The solvent and unreacted components were then distilled off under reduced pressure, obtaining 13.61 g of the product.

By ¹H-NMR spectroscopy, the compound was identified to have a structure of the formula (E). The compound had a melting point of 56°C under normal pressure.

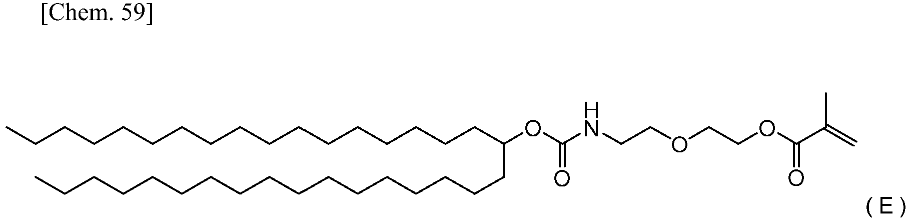

### [Synthesis Example 7] Synthesis of hydrocarbon end group-containing compound (F)

In a reactor, 10.00 g (1.92×10⁻² mol) of a compound having the formula (c): 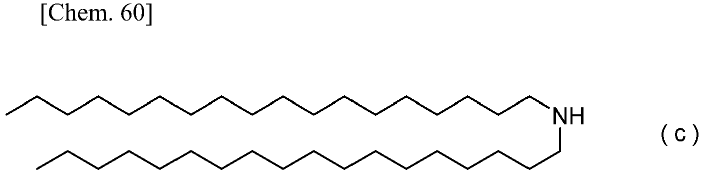 was mixed with 2.98 g (2.11×10⁻² mol) of 2-isocyanatoethyl acrylate and 50.00 g of THF, which was aged at 50°C for 12 hours. The solvent and unreacted components were then distilled off under reduced pressure, obtaining 12.11 g of the product.

By ¹H-NMR spectroscopy, the compound was identified to have a structure of the formula (F). The compound had a melting point of 38°C under normal pressure.

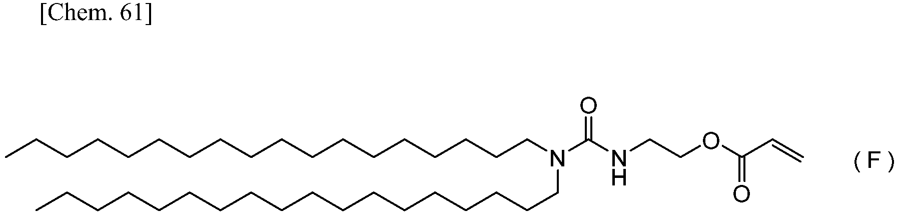

### [Synthesis Example 8] Synthesis of hydrocarbon end group-containing compound (G)

In a reactor, 10.00 g (2.83×10⁻² mol) of a compound having the formula (e): 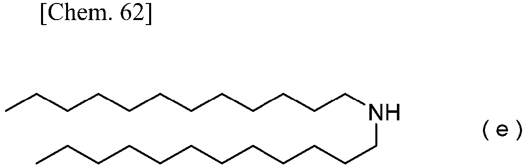 was mixed with 4.39 g (3.11×10⁻² mol) of 2-isocyanatoethyl acrylate and 50.00 g of THF, which was aged at 50°C for 12 hours. The solvent and unreacted components were then distilled off under reduced pressure, obtaining 12.72 g of the product.

By ¹H-NMR spectroscopy, the compound was identified to have a structure of the formula (G). The compound had a melting point of 21°C under normal pressure.

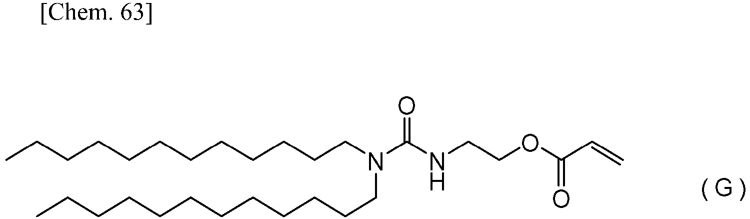

### [Synthesis Example 9] Synthesis of hydrocarbon end group-containing compound (H)

In a reactor, 10.00 g (3.07×10⁻² mol) of a compound having the formula (f): 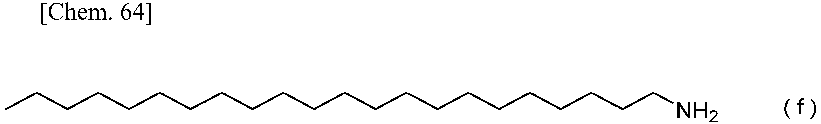 was mixed with 4.77 g (3.38×10⁻² mol) of 2-isocyanatoethyl acrylate and 50.00 g of THF, which was aged at 50°C for 12 hours. The solvent and unreacted components were then distilled off under reduced pressure, obtaining 14.32 g of the product.

By ¹H-NMR spectroscopy, the compound was identified to have a structure of the formula (H). The compound had a melting point of 48°C under normal pressure.

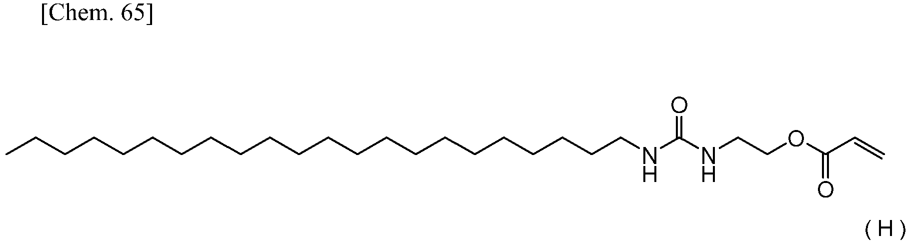

### [Synthesis Example 10] Synthesis of hydrocarbon end group-containing compound (I)

In a reactor, 10.00 g (1.92×10⁻² mol) of a compound having the formula (c): 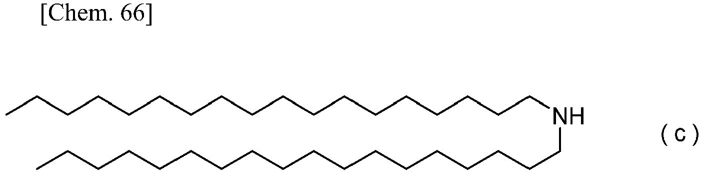 was mixed with 3.28 g (2.11×10⁻² mol) of 2-isocyanatoethyl methacrylate and 50.00 g of THF, which was aged at 50°C for 12 hours. The solvent and unreacted components were then distilled off under reduced pressure, obtaining 13.03 g of the product.

By ¹H-NMR spectroscopy, the compound was identified to have a structure of the formula (I). The compound had a melting point of 47°C under normal pressure.

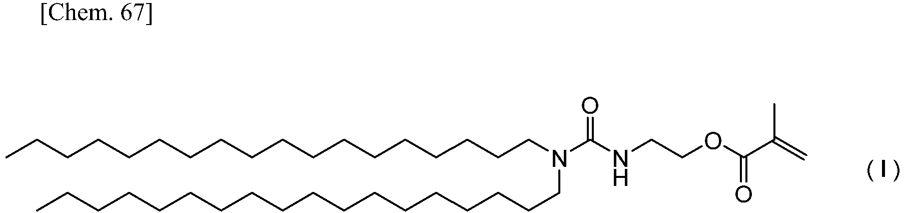

### [Synthesis Example 11] Synthesis of hydrocarbon end group-containing compound (J)

In a reactor, 10.00 g (1.92×10⁻² mol) of a compound having the formula (c): 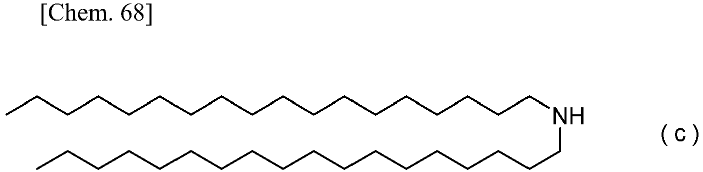 was mixed with 4.21 g (2.11 × 10⁻² mol) of 2-(2-methacryloyloxyethyloxy)ethyl isocyanate and 50.00 g of THF, which was aged at 50°C for 12 hours. The solvent and unreacted components were then distilled off under reduced pressure, obtaining 13.64 g of the product.

By ¹H-NMR spectroscopy, the compound was identified to have a structure of the formula (J). The compound had a melting point of 39°C under normal pressure.

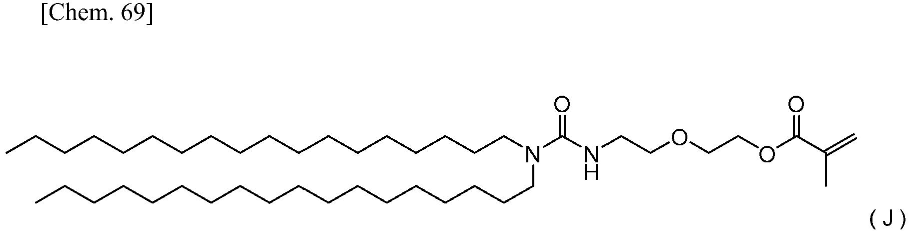

### [Synthesis Example 12] Synthesis of hydrocarbon end group-containing compound (K)

In a reactor, 10.00 g (1.92×10⁻² mol) of a compound having the formula (c): 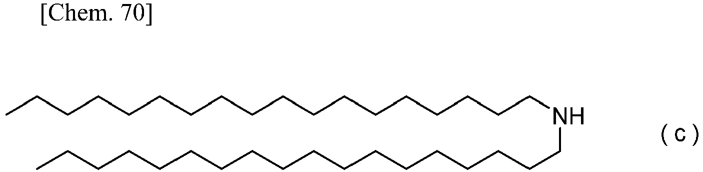 was mixed with 5.05 g (2.11×10⁻² mol) of 1,1-bis(acryloyloxymethyl)ethyl isocyanate and 50.00 g of THF, which was aged at 50°C for 12 hours. The solvent and unreacted components were then distilled off under reduced pressure, obtaining 14.50 g of the product.

By ¹H-NMR spectroscopy, the compound was identified to have a structure of the formula (K). The compound had a melting point of 20°C under normal pressure.

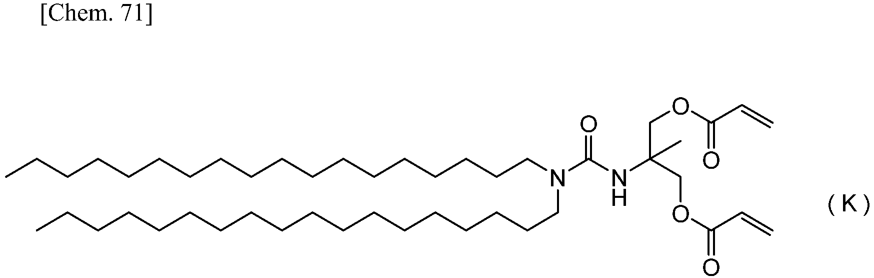

### [Comparative Synthesis Example 1] Synthesis of hydrocarbon end group-containing compound (X)

In a reactor, 10.00 g (3.70×10⁻² mol) of a compound having the formula (g): 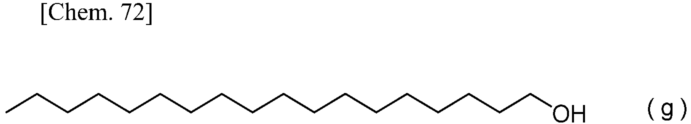 was mixed with 5.74 g (4.07×10⁻² mol) of 2-isocyanatoethyl acrylate, 50.00 g of THF, and 0.03 g of tetrakis(2-ethylhexyl) orthotitanate, which was aged at 50°C for 12 hours. The solvent and unreacted components were then distilled off under reduced pressure, obtaining 13.28 g of the product.

By ¹H-NMR spectroscopy, the compound was identified to have a structure of the formula (X). The compound had a melting point of 29°C under normal pressure.

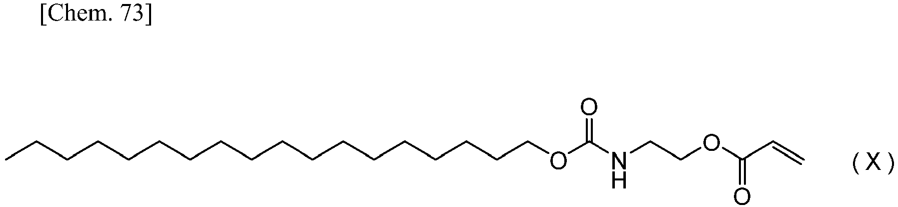

### [Comparative Synthesis Example 2] Synthesis of hydrocarbon end group-containing compound (Y)

In a reactor, 10.00 g (3.71×10⁻² mol) of a compound having the formula (h): 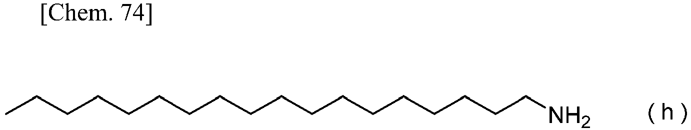 was mixed with 5.76 g (4.08×10⁻² mol) of 2- isocyanatoethyl acrylate and 50.00 g of THF, which was aged at 50°C for 12 hours. The solvent and unreacted components were then distilled off under reduced pressure, obtaining 15.01 g of the product.

By ¹H-NMR spectroscopy, the product was identified to have a structure of the formula (Y). The compound had a melting point of 42°C under normal pressure.

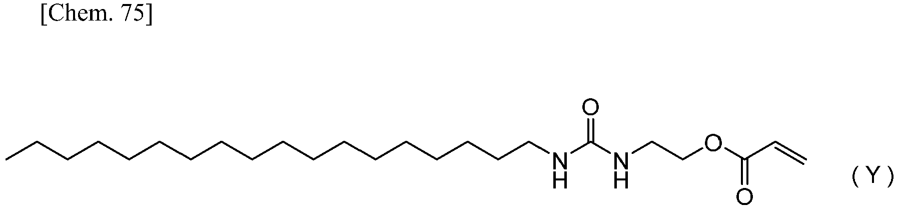

### [Examples 1 to 22 and Comparative Examples 1 to 3]

### Preparation of film-forming curable composition of actinic energy radiation-cure type

A film-forming curable composition of actinic energy radiation-cure type in solution form was prepared by blending each of Compounds (A) to (K) obtained in Synthesis Examples and Compounds (X) and (Y) obtained in Comparative Synthesis Examples in the proportion shown in Tables 1 and 2.

**[Table 1]**

| Blending amount (pbw) | Example 1 | Example 2 | Example 3 | Example 4 | Example 5 | Example 6 | Example 7 | Example 8 | Example 9 | Example 10 | Example 11 | Comp. Example 1 | Comp. Example 2 | Comp. Example 3 |
|---|---|---|---|---|---|---|---|---|---|---|---|---|---|---|
| A-9550 | 100 | 100 | 100 | 100 | 100 | 100 | 100 | 100 | 100 | 100 | 100 | 100 | 100 | 100 |
| AcOBu | 142 | 142 | 142 | 142 | 142 | 142 | 142 | 142 | 142 | 142 | 142 | 142 | 142 | 142 |
| I-184 | 3 | 3 | 3 | 3 | 3 | 3 | 3 | 3 | 3 | 3 | 3 | 3 | 3 | 3 |
| Compound A | 0.5 | | | | | | | | | | | | | |
| Compound B | | 0.5 | | | | | | | | | | | | |
| Compound C | | | 0.5 | | | | | | | | | | | |
| Compound D | | | | 0.5 | | | | | | | | | | |
| Compound E | | | | | 0.5 | | | | | | | | | |
| Compound F | | | | | | 0.5 | | | | | | | | |
| Compound G | | | | | | | 1.0 | | | | | | | |
| Compound H | | | | | | | | 0.5 | | | | | | |
| Compound I | | | | | | | | | 0.5 | | | | | |
| Compound J | | | | | | | | | | 1.0 | | | | |
| Compound K | | | | | | | | | | | 0.5 | | | |
| Compound X | | | | | | | | | | | | 0.5 | | |
| Compound Y | | | | | | | | | | | | | 0.5 | |

**[Table 2]**

| Blending amount (pbw) | Example 12 | Example 13 | Example 14 | Example 15 | Example 16 | Example 17 | Example 18 | Example 19 | Example 20 | Example 21 | Example 22 |
|---|---|---|---|---|---|---|---|---|---|---|---|
| AcOBu | 100 | 100 | 100 | 100 | 100 | 100 | 100 | 100 | 100 | 100 | 100 |
| I-184 | 0.006 | 0.006 | 0.006 | 0.006 | 0.006 | 0.006 | 0.006 | 0.006 | 0.006 | 0.006 | 0.006 |
| Compound A | 0.2 | | | | | | | | | | |
| Compound B | | 0.2 | | | | | | | | | |
| Compound C | | | 0.2 | | | | | | | | |
| Compound D | | | | 0.2 | | | | | | | |
| Compound E | | | | | 0.2 | | | | | | |
| Compound F | | | | | | 0.2 | | | | | |
| Compound G | | | | | | | 0.2 | | | | |
| Compound H | | | | | | | | 0.2 | | | |
| Compound I | | | | | | | | | 0.2 | | |
| Compound J | | | | | | | | | | 0.2 | |
| Compound K | | | | | | | | | | | 0.2 |

- A-9550:: dipentaerythritol polyacrylate (A-9550 by Shin-Nakamura Chemical Co., Ltd.)
- AcOBu:: butyl acetate
- I-184:: 1-hydroxycyclohexyl phenyl ketone (Irgacure 184 by Ciba Japan Co., Ltd.)

### Coating and preparation of cured film (1)

Each of the solutions (film-forming curable compositions of actinic energy radiation-cure type) prepared in Examples 1 to 11 and Comparative Examples 1 to 3 was applied onto a polycarbonate substrate by spin coating. After coating, nitrogen flow was passed in an inert box at room temperature for 5 minutes for solvent volatilization and leveling. Using a conveyor type metal halide UV irradiating system (Panasonic Electric Works Co., Ltd.), UV was irradiated to the coating surface in nitrogen atmosphere in an accumulative dose of 1,600 mJ/cm² to cure the composition, obtaining a cured film of 5 µm thick.

### Coating and preparation of cured film (2)

A solution was prepared by mixing 100 pbw of dipentaerythritol polyacrylate (A-9550 by Shin-Nakamura Chemical Co., Ltd.), 142 pbw of butyl acetate, and 3 pbw of 1-hydroxycyclohexyl phenyl ketone (Irgacure 184 by Ciba Japan Co., Ltd.). The solution was applied onto a polycarbonate substrate by spin coating. After coating, the coating was heated at 80°C for 1 minute for solvent volatilization and leveling. Then nitrogen flow was passed in an inert box at room temperature for 5 minutes. Using a conveyor type metal halide UV irradiating system (Panasonic Electric Works Co., Ltd.), UV was irradiated to the coating surface in air in an accumulative dose of 1,600 mJ/cm² to cure the coating, obtaining a cured film of 5 µm thick.

Each of the solutions (film-forming curable compositions of actinic energy radiation-cure type) prepared in Examples 12 to 22 was applied onto the cured film by spin coating. After coating, the coating was heated at 80°C for 1 minute for solvent volatilization and leveling. Then nitrogen flow was passed in an inert box at room temperature for 5 minutes. Using a conveyor type metal halide UV irradiating system (Panasonic Electric Works Co., Ltd.), UV was irradiated to the coating surface in nitrogen atmosphere in an accumulative dose of 1,600 mJ/cm² to cure the composition, obtaining a cured film of 5 nm thick.

The outer appearance (or transparency) of the cured film was visually observed. By the methods shown below, the cured film was measured for a contact angle with water and oleic acid as an index of antifouling, and for a contact angle with water after a wear test for the evaluation of marker ink wipe-off and wear resistance. The results are shown in Tables 3 and 4. With respect to the outer appearance (or transparency), a transparent sample is rated "Pass (○)" and a non-transparent sample is rated "Reject (×)".

### Evaluation of antifouling property

### [Measurement of contact angle with water and oleic acid]

Using a contact angle meter Drop Master (Kyowa Interface Science Co., Ltd.), the cured film was measured for contact angle with water and oleic acid under conditions: droplet volume 2 µL, temperature 25°C, and relative humidity 40%.

### [Evaluation of marker ink wipe-off]

Using a marker (large size Magic Ink by Teranishi Chemical Industry Co., Ltd.), a straight line of 3 cm long was drawn on the cured film. After drying at room temperature for 3 minutes, the marked line was wiped off with tissue paper. A sample is rated "Pass (○)" when the marks are wiped off and "Reject (×)" when not wiped off.

### Evaluation of wear resistance

### [Measurement of contact angle with water after wear test]

Using a friction wear tester (Shinto Scientific Co., Ltd.), a reciprocal wear test was performed on the surface of the cured film. After the test, a contact angle with water was measured. The rating is the average of 4 samples.

The test conditions are shown below.

| | |
|---|---|
| Rubbing member: | Bonstar steel wool #0000 (Bonstar Co., Ltd.) |
| Moving distance (single way): | 40 mm |
| Moving speed: | 4,800 mm/min |
| Load: | 300 gf/1×1 cm² |
| Wear count: | 5,000 cycles |

**[Table 3]**

| | Appearance | Water contact angle (°) | Oleic acid contact angle (°) | Marker ink wipe-off | Water contact angle after wear test (°) |
|---|---|---|---|---|---|
| Example 1 | ○ | 99 | 45 | ○ | 91 |
| Example 2 | ○ | 99 | 46 | ○ | 90 |
| Example 3 | ○ | 103 | 50 | ○ | 92 |
| Example 4 | ○ | 96 | 45 | ○ | 90 |
| Example 5 | ○ | 102 | 48 | ○ | 93 |
| Example 6 | ○ | 105 | 55 | ○ | 99 |
| Example 7 | ○ | 98 | 45 | ○ | 91 |
| Example 8 | ○ | 97 | 46 | ○ | 94 |
| Example 9 | ○ | 104 | 54 | ○ | 96 |
| Example 10 | ○ | 103 | 54 | ○ | 93 |
| Example 11 | ○ | 98 | 45 | ○ | 93 |
| Comparative Example 1 | ○ | 86 | 29 | × | 61 |
| Comparative Example 2 | ○ | 88 | 30 | × | 63 |
| Comparative Example 3 | ○ | 60 | 14 | × | not tested |

**[Table 4]**

| | Appearance | Water contact angle (°) | Oleic acid contact angle (°) | Marker ink wipe-off | Water contact angle after wear test (°) |
|---|---|---|---|---|---|
| Example 12 | ○ | 100 | 46 | ○ | 88 |
| Example 13 | ○ | 100 | 47 | ○ | 86 |
| Example 14 | ○ | 103 | 50 | ○ | 94 |
| Example 15 | ○ | 99 | 47 | ○ | 87 |
| Example 16 | ○ | 103 | 50 | ○ | 95 |
| Example 17 | ○ | 101 | 53 | ○ | 98 |
| Example 18 | ○ | 95 | 45 | ○ | 87 |
| Example 19 | ○ | 100 | 49 | ○ | 90 |
| Example 20 | ○ | 101 | 51 | ○ | 96 |
| Example 21 | ○ | 100 | 50 | ○ | 93 |
| Example 22 | ○ | 96 | 46 | ○ | 89 |

The cured films (Examples 1 to 22) of the film-forming curable compositions of actinic energy radiation-cure type using the hydrocarbon end group-containing compounds (Compounds (A) to (K)) within the scope of the invention exhibited excellent flatness, water repellent and antifouling properties. The wear test did not cause a substantial decline of water contact angle, attesting high wear resistance. In contrast, the cured films (Comparative Examples 1 and 2) of the film-forming curable compositions of actinic energy radiation-cure type using the hydrocarbon end group-containing compounds (Compounds (X) and (Y)) of different structure from the inventive hydrocarbon end group-containing compounds exhibited poor water repellent, antifouling and wear resistant properties. The cured film (Comparative Example 3) of the film-forming curable composition of actinic energy radiation-cure type in which the hydrocarbon end group-containing compound was not blended did not exhibit water repellent and antifouling properties at all.

## Claims

1. A compound which contains a hydrocarbon end group, but not fluorine in its structure, represented by the general formula (1): 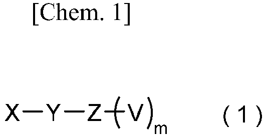 wherein X is R¹O- or R²R³N-, R¹ is a C₂₀-C₈₀ straight, branched or cyclic monovalent hydrocarbon group, R² is a C₁₀-C₄₀ straight, branched or cyclic monovalent hydrocarbon group, R³ is hydrogen or a C₁₀-C₄₀ straight, branched or cyclic monovalent hydrocarbon group, the total of carbon atoms in R² and R³ is from 20 to 80,
Y is a single bond or divalent organic group having the following structural formula:
*-C(=O)-**
*-C(=O)-O-**
*-C(=O)-NR⁴-**
*-C(=S)-NR⁴-**
wherein * designates a point of attachment to X in formula (1), ** designates a point of attachment to Z in formula (1), R⁴ is hydrogen or a C₁-C₈ straight, branched or cyclic monovalent hydrocarbon group,
Z is a single bond or C₁-C₂₀ di- to tetravalent hydrocarbon group which may contain at least one atom selected from oxygen, sulfur, nitrogen and silicon,
V is independently a C₂-C₂₀ monovalent hydrocarbon group which contains a polymerizable carbon-carbon double bond and which may contain oxygen and/or nitrogen, and
m is an integer of 1 to 3.

2. The hydrocarbon end group-containing compound of claim 1 wherein in formula (1), V has the following formula: 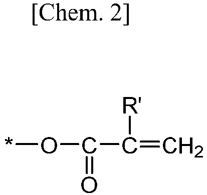 wherein * designates a point of attachment to Z in formula (1), and R' is hydrogen or a C₁-C₈ monovalent hydrocarbon group.

3. The hydrocarbon end group-containing compound of claim 1 wherein in formula (1), Y has the following structural formula:
*-C(=O)-NH-**
wherein * designates a point of attachment to X in formula (1), and ** designates a point of attachment to Z in formula (1).

4. The hydrocarbon end group-containing compound of claim 1 wherein in formula (1), m is 1.

5. The hydrocarbon end group-containing compound of claim 1 wherein in formula (1), Z is a single bond or C₁-C₁₀ divalent hydrocarbon group which may contain oxygen and/or nitrogen.

6. The hydrocarbon end group-containing compound of claim 1 which is a hydrocarbon end group-containing acrylic compound represented by the general formula (2): 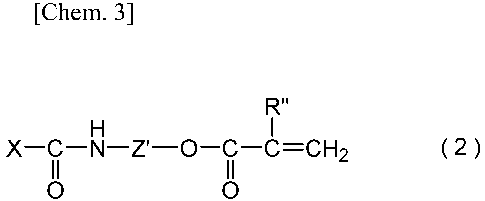 wherein X is as defined above, Z' is a C₁-C₁₀ divalent hydrocarbon group which may contain oxygen and/or nitrogen, and R" is hydrogen or methyl.

7. The hydrocarbon end group-containing compound of claim 1 which is a hydrocarbon end group-containing acrylic compound represented by the general formula (3): 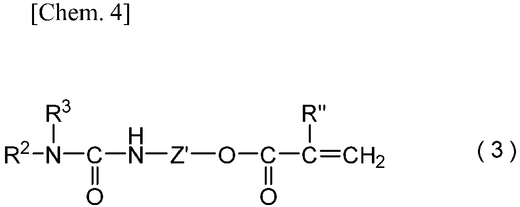 wherein R² and R³ are as defined above, Z' is a C₁-C₁₀ divalent hydrocarbon group which may contain oxygen and/or nitrogen, and R" is hydrogen or methyl.

8. The hydrocarbon end group-containing compound of claim 7 wherein in formula (3), R² and R³ each are a C₁₀-C₄₀ straight monovalent hydrocarbon group, and R² and R³ have an equal number of carbon atoms.

9. The hydrocarbon end group-containing compound of claim 1, having a melting point of at least 20°C.

10. A film-forming curable composition comprising a non-fluorinated hydrocarbon end group-containing compound having one C₂₀-C₈₀ hydrocarbon end group or two C₁₀-C₄₀ hydrocarbon end groups and at least one polymerizable group per molecule.

11. The film-forming curable composition of claim 10 wherein the non-fluorinated hydrocarbon end group-containing compound is the hydrocarbon end group-containing compound of any one of claims 1 to 9.

12. The film-forming curable composition of claim 10, further comprising a polymerization initiator.

13. The film-forming curable composition of claim 12 which is of actinic energy radiation-curable type.

14. The film-forming curable composition of claim 10, further comprising a solvent.

15. The film-forming curable composition of claim 10, further comprising a non-fluorinated acrylic compound.

16. The film-forming curable composition of claim 10, comprising fluorine-free compounds.

17. A cured film obtained by curing the film-forming curable composition of claim 10.

18. The cured film of claim 17, having a contact angle with water of at least 90° and a contact angle with oleic acid of at least 40° under conditions: droplet volume 2 µL, temperature 25°C, and relative humidity 40%.

19. An article having the cured film of claim 17 on its surface.
